# EUROPEAN PATENT SPECIFICATION

(11) **EP 4 062 176 B1**
(45) Date of publication and mention of the grant of the patent: **22.05.2024**
(21) Application number: 20829400.9
(22) Date of filing: 23.11.2020
(51) Int. Cl.: G01N 33/66, G01N 33/68

(54) **LABEL-FREE N-GLYCAN QUANTIFICATION METHODS**
MARKIERUNGSFREIE N-GLYCAN-QUANTIFIZIERUNGSVERFAHREN
PROCÉDÉS DE QUANTIFICATION DE N-GLYCANE SANS MARQUEUR

(30) Priority: 21.11.2019 US 201962938803 P
(43) Date of publication of application: 28.09.2022
(73) Proprietor: Bristol-Myers Squibb Company, Princeton, NJ 08543 (US)
(72) Inventor: CHEMMALIL, Letha, Princeton, New Jersey 08543 (US); DING, Julia, Princeton, New Jersey 08543 (US); LI, Zhengjian, Princeton, New Jersey 08543 (US)
(74) Representative: Mewburn Ellis LLP
(86) International application number: PCT/US2020/061876
(87) International publication number: WO 2021/102437

(56) References cited:
- RASMUS HANSEN ET AL: "Rapid characterization of N-linked glycans from secreted and gel-purified monoclonal antibodies using MALDI-ToF mass spectrometry", BIOTECHNOLOGY AND BIOENGINEERING, vol. 107, no. 5, 26 July 2010 (2010-07-26) , pages 902-908, XP055767111, US ISSN: 0006-3592, DOI: 10.1002/bit.22879
- Y. WADA ET AL: "Comparison of the methods for profiling glycoprotein glycans--HUPO Human Disease Glycomics/Proteome Initiative multi-institutional study", GLYCOBIOLOGY, vol. 17, no. 4, 12 January 2007 (2007-01-12), pages 411-422, XP055227506, US ISSN: 0959-6658, DOI: 10.1093/glycob/cwl086
- M GONDEK ET AL: "Purification and method optimisation for identification of carbohydrate moieties on human chorionic gonadotropin glycoforms using MALDI IT-TOF MS", FEBS OPEN BIO, vol. 9, no. S1, 1 July 2019 (2019-07-01), pages 61-61, XP055767227, US ISSN: 2211-5463, DOI: 10.1002/2211-5463.12674
- MARCELA GONDEK ET AL: "Method of optimization for identification of carbohydrate moieties on human chorionic gonadotropin glycoforms using matrix-assisted laser desorption ionization mass spectrometry", TUMOUR BIOLOGY : THE JOURNAL OF THE INTERNATIONAL SOCIETY FOR ONCODEVELOPMENTAL BIOLOGY AND MEDICINE, vol. 41, no. 7, 27 November 2018 (2018-11-27), pages 59-60, XP055767236, Switzerland ISSN: 1423-0380

## Description

### BACKGROUND OF THE DISCLOSURE

Glycosylation often plays a significant role in the biological function(s) of glycoconjugates (e.g., glycoproteins). For example, a glycoprotein's glycosylation pattern can affect its ability to fold correctly, its stability (e.g., resistance to proteolytic and/or other degradation), catalytic activity, pharmacodynamic and/or pharmacokinetic properties, and/or the ability of that glycoprotein to properly interact with other molecules. Alternatively or additionally, a glycoprotein's glycosylation pattern can affect transport and targeting of the glycoprotein, e.g., determining whether the glycoprotein remains intracellular (including, e.g., the correct targeting of the glycoprotein to the proper subcellular compartment or compartments), whether the glycoprotein will be membrane-bound and/or whether the glycoprotein will be secreted from the cell.

Monoclonal antibodies and other proteins are complex glycoproteins that are developed for treatment of various indications such as cancer and autoimmune diseases. Specifically, monoclonal antibodies are commonly glycosylated at conserved asparagine residues of the heavy chain. Glycans are important in governing the function and efficacy of the monoclonal antibody therapeutics, and as such, are generally required to be part of the critical quality attribute panel for release testing for use in humans. Depending on the terminal sugar residues, "N-linked glycans" or "N-glycans" are classified into complex, high-mannose, and hybrid N-glycans.

Hansen et al. (2010, Biotechnol. Bioeng. 107:902-908) describe workflows for glycan profiling of purified and gel-purified recombinant monoclonal antibodies, in which small-scale antibody purification and buffer exchange is combined with PNGase F glycan cleavage and graphite HyperCarb desalting. Matrix-assisted laser desorption ionization time-of-flight (MALDI-ToF) mass spectrometry (MS) was used for the detection of glycan forms. Similarly, Gondek et al. (2019, FEBS Open Bio 9(Suppl. 1):61; 2018, Tumor Biol: The journal of the international society for oncodevelopmental biology and medicine 41:59-60) isolated and detected N-linked carbohydrates attached to glycoproteins using matrix-assisted laser desorption ionization ion trap time-of-flight mass spectrometry (MALDI IT-TOF MS). Further methods for profiling glycoprotein glycans include carbon-liquid chromatography (LC)/electrospray ionization (ESI) MS, MS/MS or MALDI MS (Wada et al. (2007), Glycobiology 17(4):411-422).

Traditionally, N-linked glycans are released from the glycoprotein after denaturation and enzymatic digestion with PNGase F, followed by fluorescent labeling (such as with 2-aminobenzamide) of the liberated glycans. The labeled glycans are then separated using hydrophilic liquid chromatography (HILIC) using fluorescence detection to generate a chromatographic profile of the glycans present in the antibody samples. Despite decades of use, this process remains cumbersome, using toxic reagents and extended sample preparation time. Therefore, there are needs to efficiently quantify a glycosylation profile of a protein.

### SUMMARY OF THE DISCLOSURE

The present invention is directed to a label-free method of quantifying a glycosylation profile of a recombinant protein, comprising: (a) releasing one or more N-linked glycans from the recombinant protein by enzymatic digestion prior to the analysis; (b) separating the released N-linked glycans using a chromatography column; and (c) measuring the separated N-linked glycans by ELSD, NQAD, refractive index detector, or a charged aerosol detector (CAD). In some aspects, the recombinant protein is at least about 80%, at least about 85%, at least about 90%, at least about 95%, at least about 96%, at least about 97%, at least about 98%, at least about 99%, or about 100% pure. In some aspects, the column is a mixed mode column. In some aspects, the separation is done using one or more mobile phases. In some aspects, the column is a mixed mode porous graphite carbon (PGC) column. In some aspects, the enzyme comprises peptide N-glycosidase F (PNGaseF). In some aspects, the enzyme is incubated with the recombinant protein, wherein the one or more N-linked glycans are released from the recombinant protein prior to the separation. In some aspects, the enzyme is diluted in buffer.

In some aspects, the chromatography comprises a first mobile phase and a second mobile phase, wherein the first mobile phase and the second mobile phase are different. In some aspects, the first mobile phase comprises water. In some aspects, the second mobile phase comprises acetonitrile. In some aspects, the first mobile phase comprises formic acid (FA), Trifluoroacetic acid (TFA), Triethylamine (TEA), or any combination thereof. In some aspects, the first mobile phase comprises 0.1% FA. In some aspects, the first mobile phase comprises 0.1% TEA. In some aspects, the second mobile phase comprises formic acid (FA), Trifluoroacetic acid (TFA), Triethylamine (TEA), or any combination thereof. In some aspects, the second mobile phase comprises 0.1% FA. In some aspects, the second mobile phase comprises 0.1% TEA. In some aspects, the separation is performed at a temperature lower than 70 °C.

In some aspects, the temperature is between about 50 °C and about 70 °C, about 50 °C and about 60 °C, about 60 °C and about 70 °C, about 55 °C and about 65 °C, about 55 °C and about 60 °C, about 60 °C and about 65 °C, about 65 °C and about 70 °C, about 50 °C and about 55 °C. In some aspects, the temperature is about 50 °C, about 51 °C, about 52 °C, about 53 °C, about 54 °C, about 55 °C, about 56 °C, about 57 °C, about 58 °C, about 59 °C, about 60 °C, about 61 °C, about 62 °C, about 63 °C, about 64 °C, about 65 °C, about 66 °C, about 67 °C, about 68 °C, or about 69 °C.

In some aspects, the separation is based on a gradient. In some aspects, the gradient is from about 95% to about 5%. In some aspects, the gradient is from about 95% to about 50%, from about 95% to about 55%, from about 95% to about 60%, from about 95% to about 65%, from about 95% to about 70%, from about 95% to about 75%, from about 95% to about 80%, from about 95% to about 85%, from about 90% to about 50%, from about 90% to about 55%, from about 90% to about 60%, from about 90% to about 65%, from about 90% to about 70%, from about 90% to about 75%, from about 87% to about 50%, from about 87% to about 55%, from about 87% to about 60%, from about 87% to about 65%, from about 87% to about 70%, from about 87% to about 75%, from about 85% to about 50%, from about 85% to about 55%, from about 85% to about 60%, from about 85% to about 65%, from about 85% to about 70%, or from about 85% to about 75%. In some aspects, the gradient is from about 87% to about 75%.

In some aspects, the one or more N-glycans are Galactose (Gal), N-Acetylgalactosamine (GalNAc), Galactosamine (GalN), Glucose (Glc), N-Acetylglucosamine (GlcNAc), Glucosamine (GlcN), Mannose (Man), N-Acetylmannosamine (ManNAc), Mannosamine (ManN), Xylose (Xyl), N-Acetylneuraminic acid (Neu5Ac), N-Glycolylneuraminic acid (Neu5Gc), 2-Keto-3-deoxynononic acid (Kdn), Fucose (Fuc), Glucuronic Acid (GlcA), Iduronic acid (IdoA), Galacturonic acid (GalA), Mannuronic acid (ManA), or any combination thereof. In some aspects, the one or more N-glycans comprise one or more bi-antennary glycans. In some aspects, the bi-antennary glycans are selected from a group consisting of G0F, G0, G1F, G1, G2F, G2, S1G2F, S1G2, S2G2F, S2G2 and any combination thereof. In some aspects, the glycosylation profile comprises one or more asialylated glycans, mono-sialylated glycans, di-sialylated glycans, and/or tri-sialylated and tetra-sialylated glycans. In some aspects, the recombinant protein is an antibody. In some aspects, the antibody is an isotype selected from IgM, IgA, IgE, IgD, and IgG. In some aspects, the antibody is isotype IgG. In some aspects, the IgG antibody is selected from IgG1, IgG2, IgG3, and IgG4. In some aspects, the antibody is an antibody against GITR, an antibody against CXCR4, an antibody against CD73, an antibody against TIGIT, an antibody against OX40, an antibody against LAG3, an antibody against CSF1R, and/or an antibody against IL8. In some aspects, the antibody has a single N-linked glycosylation site. In some aspects, the single N-linked glycosylation site is Asparagine 297 (N297).

In some aspects, the recombinant protein comprises an enzyme, a hormone, a cytokine, a cell surface receptor, a protease, a cytokine receptor, or any combination thereof. In some aspects, the recombinant protein is a fusion protein. In some aspects, the fusion protein is fused to a heterologous moiety. In some aspects, the heterologous moiety is a half-life extending moiety. In some aspects, the half-life extending moiety comprises albumin, albumin binding polypeptide, a fatty acid, PAS, the β subunit of the C-terminal peptide (CTP) of human chorionic gonadotropin, polyethylene glycol (PEG), hydroxyethyl starch (HES), XTEN, albumin-binding small molecules, Fc, or a combination thereof. In some aspects, the half-life extending moiety is an Fc. In some aspects, the method is a batch release.

### BRIEF DESCRIPTION OF THE DRAWINGS

FIG. 1 shows a sample glycan chromatogram produced from a separation analysis as shown in Example 1 (mobile phase A: 0.1% formic acid in water; mobile phase B: 0.1% formic acid in acetonitrile).
FIG. 2 shows a sample glycan chromatogram produced from a separation analysis as shown in Example 2(mobile phase A: 0.1% triethylamine (TEA) in water; mobile phase B: 0.1% triethylamine (TEA) in acetonitrile).

### DETAILED DESCRIPTION OF THE DISCLOSURE

The present disclosure introduces a novel, label-free method to detect and quantify N-glycans without using fluorescent or UV labeling. Using a reversed phase porous graphite carbon column and charged aerosol detection to generate glycan profiles, the sample preparation and chromatographic separation times are greatly reduced with significant cost savings. The label-free method provides similar quantitative results to the fluorescent labeling method, confirming that this method is suitable for use in product release.

### I. Definitions

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

The use of the alternative (*e.g.,* "or") should be understood to mean either one, both, or any combination thereof of the alternatives. As used herein, the indefinite articles "a" or "an" should be understood to refer to "one or more" of any recited or enumerated component.

The terms "about" or "comprising essentially of" refer to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of" can mean within 1 or more than 1 standard deviation per the practice in the art. Alternatively, "about" or "comprising essentially of" can mean a range of up to 20%. Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one tenth and one hundredth of an integer), unless otherwise indicated.

As used herein, the term "protein of interest" is used to include any protein (either natural or recombinant), present in a mixture, for which purification is desired. Such proteins of interest include, without limitation, enzymes, hormones, growth factors, cyotokines, immunoglobulins (e.g., antibodies), and/or any fusion proteins.

As used herein, a "biomarker" is virtually any detectable compound, such as a protein, a peptide, a proteoglycan, a glycoprotein, a lipoprotein, a carbohydrate, a lipid, a nucleic acid (e.g., DNA, such as cDNA or amplified DNA, or RNA, such as mRNA), an organic or inorganic chemical, a natural or synthetic polymer, a small molecule (e.g., a metabolite), or a discriminating molecule or discriminating fragment of any of the foregoing, that is present in or derived from a biological sample, or any other characteristic that is objectively measured and evaluated as an indicator of normal biologic processes, pathogenic processes, or pharmacologic responses to a therapeutic intervention, or an indication thereof.

As used herein, the term "analyte" is used to include any molecule or protein (either natural or recombinant), present in a mixture, for which analysis or quantification is desired. Such analytes include, without limitation, small molecules, enzymes, hormones, growth factors, cytokines, immunoglobulins (e.g., antibodies), and/or any fusion proteins.

The terms "purifying," "separating," or "isolating," as used interchangeably herein, refer to increasing the degree of purity of a molecule from a composition or sample comprising the molecule and one or more impurities. Typically, the degree of purity of the molecule is increased by removing (completely or partially) at least one impurity from the composition.

As used herein, the term "mass spectrometry" refers to a sensitive technique used to detect, identify and quantitate molecules based on their mass-to-charge (m/z) ratio. Electric fields are used to separate ions according to their mass-to-charge ratio (m/z), the ratio of mass to the integer number of charges (z), as they pass along the central axis of parallel and equidistant poles or rods, such as a quadrupole, which contains four poles or rods. Each rod has two voltages applied, one of which is a fixed direct current and the second is an alternating current that cycles with a superimposed radio frequency. The magnitude of the applied electric field can be ordered such that only ions with a specific m/z ratio can travel through the quadrupole, prior to being detected. Ions with all other m/z values are deflected onto trajectories that would cause them to collide with the quadrupole rods and discharge, or be ejected from the mass analyzer field and removed via the vacuum. The quadrupole is often referred to as an exclusive detector because only ions with a specific m/z are stable in the quadrupole at any one time. Those ions with a stable trajectory are often referred to as having noncollisional, resonant or stable trajectories.

Generally, for an experiment on a triple-quadrupole mass spectrometer, the first quadrupole (Q1) is set to pass ions only of a specified m/z (precursor ions) of an expected chemical species in the sample. The second quadrupole (i.e. Q2 or the collision cell) is used to fragment the ions passing through Q1. The third quadrupole (Q3) is set to pass to the detector only ions of a specified m/z (fragment ions) corresponding to an expected fragmentation product of the expected chemical species. In some aspects, the sample is ionized in the mass spectrometer to generate one or more protonated or deprotonated molecular ions. In some aspects, the one or more protonated or deprotonated molecular are singly charged, doubly charged, triply charged or higher. In some aspects, the mass spectrometer is a triple quadrupole mass spectrometer. In some aspects, the resolutions used for Q1 and Q3 are unit resolution. In other aspects, the resolutions used for Q1 and Q3 are different. In other aspects, the resolution used for Q1 is higher than the unit resolution of Q3.

As used herein, the term "charged aerosol detection" or "CAD" refers to an analytical technique based on the nebulization of an eluent with a nitrogen (or air) carrier gas to form droplets that are then dried to remove mobile phase, producing analyte particles. The primary stream of analyte particles is met by a secondary stream that is positively charged as a result of having passed a high-voltage, platinum corona wire. The charge transfers difusionally to the opposing stream of analyte particles, and is further transferred to a collector where it is measured by a highly sensitive electrometer, generating a signal in direct proportion to the quantity of analyte present.

As used herein, the term "fluorophore" refers to a fluorescent chemical compound that can re-emit light upon light excitation. Fluorophores typically contain several combined aromatic groups, or planar or cyclic molecules with several π bonds. Two commonly used fluorophores are 2-AB (2-aminobenzamide) and 2-AA (anthranilic acid or 2-aminobenzoic acid). Other fluorophores include PA (2-Aminopyridine), AMAC (2-aminoacridone), ANDS (7-Amino-1,3-naphthalenedisulfonic acid), ANTS (8-Aminonaphthalene-1,3,6-trisulfonic acid), APTS (9-Aminopyrene-1,4,6-trisulfonic acid), and 3-(acetylamino)-6-aminoacridine.

A "glycan profile" as used in the disclosure should be understood to be any defined set of values of quantitative results for glycans that can be used for comparison to reference values or profiles derived from another sample or a group of samples. For instance, a glycan profile of a sample from a protein sample might be significantly different from a glycan profile of a sample from an alternate source. A glycan profile can aid in predicting or anticipating a protein's pharmacodynamic (PD) or pharmacokinetic(PK) therapeutic effects by comparing the profile to a reference or standard profile. Reference and sample glycan profiles can be generated by any analysis instrument capable of detecting glycans, such as a charged aerosol detector (CAD).

The term "chromatography" refers to any kind of technique which separates a protein of interest (e.g., an antibody) from other molecules (e.g., contaminants) present in a mixture. Usually, the protein of interest is separated from other molecules (e.g., contaminants) as a result of differences in rates at which the individual molecules of the mixture migrate through a stationary medium under the influence of a moving phase, or in bind and elute processes. The term "matrix" or "chromatography matrix" are used interchangeably herein and refer to any kind of sorbent, resin or solid phase which in a separation process separates a protein of interest (e.g., an Fc region containing protein such as an immunoglobulin) from other molecules present in a mixture. Non-limiting examples include particulate, monolithic or fibrous resins as well as membranes that can be put in columns or cartridges. Examples of materials for forming the matrix include polysaccharides (such as agarose and cellulose); and other mechanically stable matrices such as silica (e.g. controlled pore glass), poly(styrenedivinyl)benzene, polyacrylamide, ceramic particles and derivatives of any of the above. Examples for typical matrix types suitable for the method of the present disclosure are cation exchange resins, affinity resins, anion exchange resins or mixed mode resins. A "ligand" is a functional group that is attached to the chromatography matrix and that determines the binding properties of the matrix. Examples of "ligands" include, but are not limited to, ion exchange groups, hydrophobic interaction groups, hydrophilic interaction groups, thiophilic interactions groups, metal affinity groups, affinity groups, bioaffinity groups, and mixed mode groups (combinations of the aforementioned). Some preferred ligands that can be used herein include, but are not limited to, strong cation exchange groups, such as sulphopropyl, sulfonic acid; strong anion exchange groups, such as trimethylammonium chloride; weak cation exchange groups, such as carboxylic acid; weak anion exchange groups, such as N5N diethylamino or DEAE; hydrophobic interaction groups, such as phenyl, butyl, propyl, hexyl; and affinity groups, such as Protein A, Protein G, and Protein L. In order that the present disclosure can be more readily understood, certain terms are first defined. As used in this application, except as otherwise expressly provided herein, each of the following terms shall have the meaning set forth below. Additional definitions are set forth throughout the application.

The term "affinity chromatography" refers to a protein separation technique in which a protein of interest (e.g., an Fc region containing protein of interest or antibody) is specifically bound to a ligand which is specific for the protein of interest. Such a ligand is generally referred to as a biospecific ligand. In some aspects, the biospecific ligand (e.g., Protein A or a functional variant thereof) is covalently attached to a chromatography matrix material and is accessible to the protein of interest in solution as the solution contacts the chromatography matrix. The protein of interest generally retains its specific binding affinity for the biospecific ligand during the chromatographic steps, while other solutes and/or proteins in the mixture do not bind appreciably or specifically to the ligand. Binding of the protein of interest to the immobilized ligand allows contaminating proteins or protein impurities to be passed through the chromatography matrix while the protein of interest remains specifically bound to the immobilized ligand on the solid phase material. The specifically bound protein of interest is then removed in active form from the immobilized ligand under suitable conditions (e.g., low pH, high pH, high salt, competing ligand etc.), and passed through the chromatographic column with the elution buffer, free of the contaminating proteins or protein impurities that were earlier allowed to pass through the column. Any component can be used as a ligand for purifying its respective specific binding protein, e.g., antibody. However, in various methods according to the present disclosure, Protein A is used as a ligand for an Fc region containing a target protein. The conditions for elution from the biospecific ligand (e.g., Protein A) of the target protein (e.g., an Fc region containing protein) can be readily determined by one of ordinary skill in the art. In some aspects, Protein G or Protein L or a functional variant thereof can be used as a biospecific ligand. In some aspects, a biospecific ligand such as Protein A is used at a pH range of 5-9 for binding to an Fc region containing protein, washing or re-equilibrating the biospecific ligand/target protein conjugate, followed by elution with a buffer having pH above or below 4 which contains at least one salt.

The term "buffer" as used herein, refers to a substance which, by its presence in solution, increases the amount of acid or alkali that must be added to cause unit change in pH. A buffered solution resists changes in pH by the action of its acid-base conjugate components. Buffered solutions for use with biological reagents are generally capable of maintaining a constant concentration of hydrogen ions such that the pH of the solution is within a physiological range. Traditional buffer components include, but are not limited to, organic and inorganic salts, acids and bases.

The term "conductivity" as used herein, refers to the ability of an aqueous solution to conduct an electric current between two electrodes. In solution, the current flows by ion transport. Therefore, with an increasing amount of ions present in the aqueous solution, the solution will have a higher conductivity. The unit of measurement for conductivity is milliSiemens per centimeter (mS/cm), and can be measured using a conductivity meter.

The term "mobile phase" as used herein refers to the liquid or gas that flows through a chromatography system, moving the materials to be separated at different rates over the stationary phase. A mobile phase can be polar or non-polar. Polar mobile phases are commonly employed in connection with non-polar stationary phase, and these chromatography separations are known as reversed phase chromatography. Conversely, non-polar mobile phases are often employed in connection with polar stationary phases, and are commonly known as normal phase chromatography.

The term "stationary phase" as used herein refers to the solid or liquid phase of a chromatography system on which the materials to be separated are selectively adsorbed. Commonly, a silica is used as a stationary phase.

The term "chromatography column" or "column" in connection with chromatography as used herein, refers to a container, frequently in the form of a cylinder or a hollow pillar which is filled with the chromatography matrix or resin. The chromatography matrix or resin is the material which provides the physical and/or chemical properties that are employed for purification.

The terms "ion-exchange" and "ion-exchange chromatography" refer to a chromatographic process in which an ionizable solute of interest (e.g., a protein of interest in a mixture) interacts with an oppositely charged ligand linked (e.g., by covalent attachment) to a solid phase ion exchange material under appropriate conditions of pH and conductivity, such that the solute of interest interacts non-specifically with the charged compound more or less than the solute impurities or contaminants in the mixture. The contaminating solutes in the mixture can be washed from a column of the ion exchange material or are bound to or excluded from the resin, faster or slower than the solute of interest. "Ion-exchange chromatography" specifically includes cation exchange (CEX), anion exchange (AEX), and mixed mode chromatography.

A "cation exchange resin" or "cation exchange membrane" refers to a solid phase which is negatively charged, and which has free anions for exchange with cations in an aqueous solution passed over or through the solid phase. Any negatively charged ligand attached to the solid phase suitable to form the cation exchange resin can be used, e.g., a carboxylate, sulfonate and others as described below. Commercially available cation exchange resins include, but are not limited to, for example, those having a sulfonate based group (e.g., MonoS, MiniS, Source 15S and 30S, SP SEPHAROSE^{®} Fast Flow, SP SEPHAROSE^{®} High Performance, Capto S, Capto SP ImpRes from GE Healthcare, TOYOPEARL^{®} SP-650S and SP-650M from Tosoh, MACRO-PREP^{®} High S from BioRad, Ceramic HyperD S, TRISACRYL^{®} M and LS SP and Spherodex LS SP from Pall Technologies); a sulfoethyl based group (e.g., FRACTOGEL^{®} SE, from EMD, POROS^{®} S-10 and S-20 from Applied Biosystems); a sulphopropyl based group (e.g., TSK Gel SP 5PW and SP-5PW-HR from Tosoh, POROS^{®} HS-20, HS 50, and POROS^{®} XS from Life Technologies); a sulfoisobutyl based group (e.g., FRACTOGEL^{®} EMD SO₃⁻ from EMD); a sulfoxyethyl based group (e.g., SE52, SE53 and Express-Ion S from Whatman), a carboxymethyl based group (e.g., CM SEPHAROSE^{®} Fast Flow from GE Healthcare, Hydrocell CM from Biochrom Labs Inc., MACRO-PREP^{®} CM from BioRad, Ceramic HyperD CM, TRISACRYL^{®} M CM, TRISACRYL^{®} LS CM, from Pall Technologies, Matrx CELLUFINE^{®} C500 and C200 from Millipore, CM52, CM32, CM23 and Express-Ion C from Whatman, TOYOPEARL^{®} CM-650S, CM-650M and CM-650C from Tosoh); sulfonic and carboxylic acid based groups (e.g., BAKERBOND^{®} Carboxy-Sulfon from J. T. Baker); a carboxylic acid based group (e.g., WP CBX from J. T Baker, DOWEX^{®}. MAC-3 from Dow Liquid Separations, AMBERLITE^{®} Weak Cation Exchangers, DOWEX^{®} Weak Cation Exchanger, and DIAION^{®} Weak Cation Exchangers from Sigma-Aldrich and FRACTOGEL^{®} EMD COO-- from EMD); a sulfonic acid based group (e.g., Hydrocell SP from Biochrom Labs Inc., DOWEX^{®} Fine Mesh Strong Acid Cation Resin from Dow Liquid Separations, UNOsphere S, WP Sulfonic from J. T. Baker, SARTOBIND^{®} S membrane from Sartorius, AMBERLITE^{®} Strong Cation Exchangers, DOWEX^{®} Strong Cation and DIAION^{®} Strong Cation Exchanger from Sigma-Aldrich); or a orthophosphate based group (e.g., P11 from Whatman). Other cation exchange resins include carboxy-methyl-cellulose, BAKERBOND ABXTM, Ceramic HyperD Z, Matrex Cellufine C500, Matrex Cellufine C200.

An "anion exchange resin" or "anion exchange membrane" refers to a solid phase which is positively charged, thus having one or more positively charged ligands attached thereto. Any positively charged ligand attached to the solid phase suitable to form the anionic exchange resin can be used, such as quaternary amino groups. Commercially available anion exchange resins include DEAE cellulose, POROS^{®} PI 20, PI 50, HQ 10, HQ 20, HQ 50, D 50 from Applied Biosystems, SARTOBIND^{®} Q from Sartorius, MonoQ, MiniQ, Source 15Q and 30Q, Q, DEAE and ANX SEPHAROSE^{®} Fast Flow, Q SEPHAROSE^{®} High Performance, QAE SEPHADEX^{®} and FAST Q SEPHAROSE^{®} (GE Healthcare), WP PEI, WP DEAM, WP QUAT from J. T. Baker, Hydrocell DEAE and Hydrocell QA from Biochrom Labs Inc., UNOsphere Q, MACRO-PREP^{®}. DEAE and MACRO-PREP^{®} High Q from Biorad, Ceramic HyperD Q, ceramic HyperD DEAE, TRISACRYL^{®} M and LS DEAE, Spherodex LS DEAE, QMA SPHEROSIL^{®} LS, QMA SPHEROSIL^{®}. M and MUSTANG^{®} Q from Pall Technologies, DOWEX^{®} Fine Mesh Strong Base Type I and Type II Anion Resins and DOWEX^{®} MONOSPHER E 77, weak base anion from Dow Liquid Separations, INTERCEPT^{®} Q membrane, Matrex CELLUFINE^{®} A200, A500, Q500, and Q800, from Millipore, FRACTOGEL^{®} EMD TMAE, FRACTOGEL^{®} EMD DEAE and FRACTOGEL^{®} EMD DMAE from EMD, AMBERLITE^{®} weak strong anion exchangers type I and II, DOWEX^{®} weak and strong anion exchangers type I and II, DIAION^{®} weak and strong anion exchangers type I and II, DUOLITE^{®} from Sigma-Aldrich, TSK gel Q and DEAE 5PW and 5PW-HR, TOYOPEARL^{®} SuperQ-650S, 650M and 650C, QAE-550C and 650S, DEAE-650M and 650C from Tosoh, QA52, DE23, DE32, DE51, DE52, DE53, Express-Ion D or Express-Ion Q from Whatman, and SARTOBIND^{®} Q (Sartorius Corporation, New York, USA). Other anion exchange resins include POROS XQ, SARTOBIND^{®} Q, Q SEPHAROSE^{™} XL, Q SEPHAROSE^{™} big beads, DEAE Sephadex A-25, DEAE Sephadex A-50, QAE Sephadex A-25, QAE Sephadex A-50, Q SEPHAROSE^{™} high performance, Q SEPHAROSE^{™} XL, Resource Q, Capto Q, Capto DEAE, Toyopearl GigaCap Q, Fractogel EMD TMAE HiCap, Nuvia Q, or PORGS PI.

A "porous graphite carbon (PGC) column" or "porous graphite carbon (PGC) reversed phase column" exhibits unique properties as a stationary phase, offering retention of very polar analytes and separation of structurally-related substances. On a microscopic scale, the surface of PGC is composed of flat sheets of hexagonally arranged carbon atoms as in a very large polynuclear aromatic molecule. The surface is crystalline and highly reproducible, with no micropores or chemically bonded phase. The properties of this column as a stationary phase in HPLC can be utilized to provide solutions to a wide range of what might normally be considered problematic separations in HPLC. This column provides unique retention and separation of very polar compounds, and the surface is stereo-selective with the capability to separate geometric isomers and other closely related compounds. PGC provides retention for native glycan species. Separation on PGC is facilitated by the combination of reverse phase behavior, based on the hydrophobicity of analysis, and a polar retention effect of graphite, based on the high polarizability of graphite carbon material. PGC is sensitive to structural isomers as well as linkage isomers and is able to resolve isomeric glycans. One example of a porous graphite carbon column is HYPERCARB^{™} (THERMO SCIENTIFIC^{™}).

As used herein "N-linked glycan" refers to a protein modification where a glycan is linked to a glycoconjugate via a nitrogen linkage. The acceptors of the glycan are selected asparagine residues of polypeptide chains that have entered the periplasm or the lumen of the ER, respectively. Oligosaccharyltransferase, the central enzyme of the N-glycosylation pathway, catalyses the formation of an N-glycosidic linkage of the oligosaccharide to the side-chain amide of asparagine residues that are specified by the consensus sequence N-X-S/T, where X can be any amino acid residue. All eukaryotic N-glycans share a common core sequence, Manα1-3(Manα1-6)Manβ1-4GlcNAcβ1-4GlcNAcβ1-Asn-X-Ser/Thr, and are classified into three types: (1) oligomannose, in which only Man residues extend the core; (2) complex, in which "antennae" initiated by GlcNAc extend the core; and (3) hybrid, in which Man extends the Manα1-6 arm of the core and one or two GlcNAcs extend the Manα1-3 arm.

As used herein, "N-linked glycosylation" refers the attachment of oligosaccharides to a nitrogen atom, usually the N4 of asparagine residues. N-glycosylation can occur on secreted or membrane bound proteins, mainly in eukaryotes and archaea.

As used herein the term "contaminant" is used to cover any undesired component or compound within a mixture. In cell cultures, cell lysates, or clarified bulk (e.g., clarified cell culture supernatant), contaminants include, for example, host cell nucleic acids (e.g., DNA) and host cell proteins present in a cell culture medium. Host cell contaminant proteins include, without limitation, those naturally or recombinantly produced by the host cell, as well as proteins related to or derived from the protein of interest (e.g., proteolytic fragments) and other process related contaminants. In certain aspects, the contaminant precipitate is separated from the cell culture using another means, such as centrifugation, sterile filtration, depth filtration and tangential flow filtration.

The term "antibody" refers, in some aspects, to a protein comprising at least two heavy (H) chains and two light (L) chains inter-connected by disulfide bonds. Each heavy chain is comprised of a heavy chain variable region (abbreviated herein as VH) and a heavy chain constant region (abbreviated herein as CH). In some antibodies, e.g., naturally-occurring IgG antibodies, the heavy chain constant region is comprised of a hinge and three domains, CH1, CH2 and CH3. In some antibodies, e.g., naturally-occurring IgG antibodies, each light chain is comprised of a light chain variable region (abbreviated herein as VL) and a light chain constant region. The light chain constant region is comprised of one domain (abbreviated herein as CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, and FR4. The variable regions of the heavy and light chains contain a binding domain that interacts with an antigen. A heavy chain can have the C-terminal lysine or not. The term "antibody" can include a bispecific antibody or a multispecific antibody.

An "IgG antibody", e.g., a human IgG1, IgG2, IgG3 and IgG4 antibody, as used herein has, in some aspects, the structure of a naturally-occurring IgG antibody, i.e., it has the same number of heavy and light chains and disulfide bonds as a naturally-occurring IgG antibody of the same subclass. For example, an IgG1, IgG2, IgG3 or IgG4 antibody can consist of two heavy chains (HCs) and two light chains (LCs), wherein the two HCs and LCs are linked by the same number and location of disulfide bridges that occur in naturally-occurring IgG1, IgG2, IgG3 and IgG4 antibodies, respectively (unless the antibody has been mutated to modify the disulfide bridges).

An immunoglobulin can be from any of the commonly known isotypes, including but not limited to IgA, secretory IgA, IgG and IgM. The IgG isotype is divided in subclasses in certain species: IgG1, IgG2, IgG3 and IgG4 in humans, and IgG1, IgG2a, IgG2b and IgG3 in mice. Immunoglobulins, e.g., IgG1, exist in several allotypes, which differ from each other in at most a few amino acids. "Antibody" includes, by way of example, both naturally-occurring and non-naturally-occurring antibodies; monoclonal and polyclonal antibodies; chimeric and humanized antibodies; human and nonhuman antibodies and wholly synthetic antibodies.

The term "antigen-binding portion" of an antibody, as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) a Fab fragment (fragment from papain cleavage) or a similar monovalent fragment consisting of the VL, VH, LC and CH1 domains; (ii) a F(ab')2 fragment (fragment from pepsin cleavage) or a similar bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; (vi) an isolated complementarity determining region (CDR) and (vii) a combination of two or more isolated CDRs which can optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see, e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

The term "recombinant human antibody," as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences.

As used herein, "isotype" refers to the antibody class (e.g., IgG1, IgG2, IgG3, IgG4, IgM, IgA1, IgA2, IgD, and IgE antibody) that is encoded by the heavy chain constant region genes.

Amino acids are referred to herein by either their commonly known three letter symbols or by the one-letter symbols recommended by the IUPAC-IUB Biochemical Nomenclature Commission. Nucleotides, likewise, are referred to by their commonly accepted single-letter codes.

As used herein, the term "polypeptide" refers to a molecule composed of monomers (amino acids) linearly linked by amide bonds (also known as peptide bonds). The term "polypeptide" refers to any chain or chains of two or more amino acids, and does not refer to a specific length of the product. As used herein the term "protein" is intended to encompass a molecule comprised of one or more polypeptides, which can in some instances be associated by bonds other than amide bonds. On the other hand, a protein can also be a single polypeptide chain. In this latter instance the single polypeptide chain can in some instances comprise two or more polypeptide subunits fused together to form a protein. The terms "polypeptide" and "protein" also refer to the products of post-expression modifications, including without limitation glycosylation, acetylation, phosphorylation, amidation, derivatization by known protecting/blocking groups, proteolytic cleavage, or modification by non-naturally occurring amino acids. A polypeptide or protein can be derived from a natural biological source or produced by recombinant technology, but is not necessarily translated from a designated nucleic acid sequence. It can be generated in any manner, including by chemical synthesis.

As used herein, the term "abatacept" refers to a genetically engineered fusion protein, which consists of the functional binding domain of human Cytotoxic T-Lymphocyte Antigen-4 (CTLA-4) and the Fc domain of human monoclonal immunoglobulin, of the IgG1 class. Abatacept is comprised of 2 homologous glycosylated polypeptide chains of approximately 46 kDa each which are covalently linked through a single disulfide bond.

As used herein, the term "belatacept" refers to a mutant CTLA4-Fc polypeptide that comprises two mutations relative to abatacept: the amino acid at position 29 is mutated to tyrosine and the amino acid at position 104 is mutated to glutamate. In some embodiments, for example, a beta polypeptide comprises at least the amino acid sequence of the extracellular domain of CTLA-4^{A29YL104E}-Fc. Non-limiting examples of belatacept include SEQ ID NO: 4. For example, belatacept are further described in U.S. Provisional Application No. US 2009-0252749 A1, published October 8, 2009.

The terms "polynucleotide" or "nucleotide" as used herein are intended to encompass a singular nucleic acid as well as plural nucleic acids, and refers to an isolated nucleic acid molecule or construct, e.g., messenger RNA (mRNA), complementary DNA (cDNA), or plasmid DNA (pDNA). In certain aspects, a polynucleotide comprises a conventional phosphodiester bond or a non-conventional bond (e.g., an amide bond, such as found in peptide nucleic acids (PNA)).

The term "nucleic acid" refers to any one or more nucleic acid segments, e.g., DNA, cDNA, or RNA fragments, present in a polynucleotide. When applied to a nucleic acid or polynucleotide, the term "isolated" refers to a nucleic acid molecule, DNA or RNA, which has been removed from its native environment, for example, a recombinant polynucleotide encoding an antigen binding protein contained in a vector is considered isolated for the purposes of the present disclosure. Further examples of an isolated polynucleotide include recombinant polynucleotides maintained in heterologous host cells or purified (partially or substantially) from other polynucleotides in a solution. Isolated RNA molecules include in vivo or in vitro RNA transcripts of polynucleotides of the present disclosure. Isolated polynucleotides or nucleic acids according to the present disclosure further include such molecules produced synthetically. In addition, a polynucleotide or a nucleic acid can include regulatory elements such as promoters, enhancers, ribosome binding sites, or transcription termination signals.

Various aspects of the disclosure are described in further detail in the following subsections.

### II. Methods of Glycan Release and Separation

The present disclosure is directed to methods of analyzing glycan, e.g., N-linked glycan, in a protein. All eukaryotic N-glycans share a common core sequence, Manα1-3(Manα1-6)Manβ1-4GlcNAcβ1-4GlcNAcβ1-Asn-X-Ser/Thr, and are classified into three types: (1) oligomannose, in which only Man residues extend the core; (2) complex, in which "antennae" initiated by GlcNAc extend the core; and (3) hybrid, in which Man extends the Manα1-6 arm of the core and one or two GlcNAcs extend the Manα1-3 arm. After initial processing in the endoplasmic reticulum, glycoproteins are transported to the Golgi where further processing can take place. Trimmed N-linked oligosaccharide chains can be modified by addition of several mannose residues, resulting in a "high-mannose oligosaccharide." Alternatively or additionally, one or more monosaccharide units of N-acetylglucosamine can be added to the core mannose subunits to form "complex oligosaccharides." Galactose can be added to N-acetylglucosamine subunits, and sialic acid subunits can be added to galactose subunits, resulting in chains that terminate with any of a sialic acid, a galactose, or an N-acetylglucosamine residue. A fucose residue can be added to an N-acetylglucosamine residue of the core oligosaccharide. Each of these additions is catalyzed by specific glycosyl transferases.

Hybrid glycans comprise characteristics of both high-mannose and complex glycans. For example, one branch of a hybrid glycan can comprise primarily or exclusively mannose residues, while another branch can comprise N-acetylglucosamine, sialic acid, galactose, and/or fucose sugars. N-linked glycans are involved in a variety of cellular processes. For example, N-linked glycans contribute to proper protein folding in eukaryotic cells. Chaperone proteins in the endoplasmic reticulum (e.g., calnexin and calreticulin) bind to the three glucose residues present on the N-linked glycan core. Chaperone proteins typically aid in the folding of the protein to which the glycan is attached. Following proper folding, the three glucose residues are removed, and the N-linked glycan can move on to further processing reactions. If the protein fails to fold properly, the three glucose residues are reattached, allowing the protein to re-associate with chaperones. This cycle can repeat several times until a protein reaches it proper conformation. If a protein repeatedly fails to properly fold, it is usually excreted from the endoplasmic reticulum and degraded by cytoplasmic proteases. Alternatively or additionally, N-linked glycans contribute to protein folding by steric effects. For example, cysteine residues in a peptide can be temporarily blocked from forming disulfide bonds with other cysteine residues, due to the size of a nearby glycan. Presence of an N-linked glycan, therefore, can allow a cell to control which cysteine residues will form disulfide bonds. The initial oligosaccharide chain is usually trimmed by specific glycosidase enzymes in the endoplasmic reticulum, resulting in a short, branched core oligosaccharide composed of two N-acetylglucosamine and three mannose residues.

N-linked glycans can be involved in cell-cell interactions. For example, tumor cells frequently produce abnormal N-glycan structures, which can be recognized by the CD337 receptor on natural killer cells as a sign that the cell in question is cancerous. N-linked glycans can be involved in targeting of degradative lysosomal enzymes to the lysosome. In particular, modification of an N-linked glycan with a mannose-6-phosphate residue can serve as a signal that the protein to which this glycan is attached should be targeted to the lysosome. Thus, the present disclosure encompasses the recognition that it is important to determine the glycosylation pattern of N-linked glycans (e.g., N-linked glycans which are conjugated to glycoproteins). Methods described herein can be used to analyze the characteristics (e.g., composition and/or structure) of any N-linked glycans.

The first step in glycan analysis of glyconjugates such as glycoproteins is the release of the sugars from the molecules to which they are attached. N-linked glycans on a glycoprotein can be released by an amidase such as peptide-N-glycosidase F (PNGase F).

Glycans are commonly derivatized with labels such as a fluorophore to enhance their analysis. Traditionally, mass spectrometry or fluorescence detector was the technique used to analyze release glycans that have been labeled with fluorescent compounds such as 2-AB. Mass spectrometry ("MS" or "mass-spec") is an analytical technique used to measure the mass-to-charge ratio ions. This is achieved by ionizing the sample and separating ions of differing masses and recording their relative abundance by measuring intensities of ion flux. A typical mass spectrometer comprises three parts: an ion source, a mass analyzer, and a detector system. The ion source is the part of the mass spectrometer that ionizes the substance under analysis (the analyte). The ions are then transported by magnetic or electric fields to the mass analyzer that separates the ions according to their mass-to-charge ratio (m/z). Many mass spectrometers use two or more mass analyzers for tandem mass spectrometry (MS/MS). The detector records the charge induced or current produced when an ion passes by or hits a surface. A mass spectrum is the result of measuring the signal produced in the detector when scanning m/z ions with a mass analyzer. However, the present invention is directed to a method of analyzing a glycan profile without using a label and without mass spectrometry. Charged aerosol detection (CAD) is essentially a three-step process: First, the detector converts the analyte molecules eluting from the column into dry particles. The number of particles increases proportionally with the amount of analyte. Secondly, a stream of positively-charged gas collides with the analyte particles. The charge is then transferred to the particles-the larger the particles, the greater the charge. Finally, the particles are transferred to a collector where the charge is measured by a highly-sensitive electrometer.

The methods of the present disclosure are useful for releasing and separating N-linked glycans without derivatization. The present invention is directed to a label-free method of quantifying a glycosylation profile of a recombinant protein, comprising (a) releasing one or more N-linked glycans from the recombinant protein by an enzyme prior to the analysis; (b) separating the released N-linked glycans using a chromatography column; and (c) measuring the separated N-linked glycans by ELSD, NQAD, refractive index detector, or a charged aerosol detector (CAD). In some aspects, methods described herein can provide for one or more of glycoconjugate digestion and/or glycan release, fluorescent labeling, and glycan purification for subsequent analysis. Methods of analyzing compounds from biological sources often include a derivatization step to introduce a fluorophore that facilitates detection after chromatographic separation. In one aspect, a label is used to tag release glycans prior to analysis. In various aspects of the reaction mixture, the N-glycan is linked to the label via the reducing end of the N-glycan. One type of label is a fluorophore, which is a molecule that absorbs the light of one wavelength and emits another wavelength. In some aspects, the label is a fluorophore. The method according to the invention is label-free.

The methods of the present disclosure are useful to release N-glycans that are linked to proteins at asparagine residue sites. In some aspects, the enzyme comprises peptide N-glycosidase F (PNGaseF). PNGaseF is an enzyme, commonly derived from *Elizabethkingia miricola* bacteria, that cleaves an entire glycan from a glycoprotein, and requires that the glycosylated asparagine moiety be substituted on its amino (R1) and carboxyl (R2) terminus with a polypeptide chain. The PNGase F protein sequence can be found at UniProt ID: P21163. Natural variants of the PNGaseF enzyme are known in the art. For example, natural variants of PNGaseF can contain one or more amino acid substitutions selected from D100N, E158Q, E246Q, or a combination thereof. The methods of the present disclosure can also be achieved using a functional fragment of a PNGaseF enzyme to release N-glycans from asparagine residues.

The methods of the present disclosure also contemplate the use of other enzymes to release N-Glycans from amino acids. In some aspects, the enzyme can be endoglycosidase F1, endoglycosidase F2, endoglycosidase F3, endoglycosidase H, or a functional fragment thereof. In some aspects, the enzyme is endoglycosidase F1. In some aspects, the enzyme is endoglycosidase F2. In some aspects, the enzyme is endoglycosidase F3. In some aspects, the enzyme is endoglycosidase H. In other aspects, more than one enzyme can be used to cleave the N-glycans.

The methods of the present disclosure are useful to further separate N-glycans from the proteins and from each other. The method of the present invention comprises separation of one or more N-linked glycans during a chromatography comprising a column. In some aspects, the column is a hydrophilic interaction liquid chromatography (HILIC)-UPLC. In some aspects, the column is a reversed phase (RP)-UPLC column. Other examples for typical matrix types suitable for the columns in the methods of the present disclosure are cation exchange resins, affinity resins, anion exchange resins or mixed mode resins. Other binding ligands can be attached to the chromatography resin to alter the binding properties of the column. Examples include, but are not limited to, ion exchange groups, hydrophobic interaction groups, hydrophilic interaction groups, thiophilic interactions groups, metal affinity groups, affinity groups, bioaffinity groups, and mixed mode groups (combinations of the aforementioned). The methods of the present disclosure also can be used to separate N-glycans using electrophoresis. Electrophoresis has been used for the separation and analysis of mixtures. Electrophoresis involves the migration and separation of molecules in an electric field based on differences in mobility. Various forms of electrophoresis are known, including free zone electrophoresis, gel electrophoresis, isoelectric focusing, and capillary electrophoresis. Capillary electrophoresis (CE), is directed to the separation of free and bound label. In general, CE involves introducing a sample into a capillary tube. Since each of the sample constituents has its own individual electrophoretic mobility, those with greater mobility travel through the capillary tube faster than those with slower mobility. Therefore, the components of the sample are resolved into discrete zones in the capillary tube during their migration through the tube. In some aspects, the electrophoresis is capillary electrophoresis. In some aspects, the electrophoresis is gel electrophoresis.

The methods of the present disclosure are useful to analyze N-glycans after separation using a chromatography comprising a column. In some aspects, the method comprises using mixed-mode chromatography. Mixed-mode chromatography (MMC), or multimodal chromatography, refers to chromatographic methods that utilize more than one form of interaction between the stationary phase and analytes in order to achieve their separation, which is distinct from conventional single-mode chromatography that separates components based on one critical attribute. In some aspects, the column is a mixed mode column. In some aspects, the separation is done using one or more mobile phases. In some aspects, the column is a mixed mode porous graphite carbon (PGC) column. In some aspects, the disclosure provides a method of measuring a glycosylation profile of a recombinant protein comprising (i) releasing one or more N-linked glycans using an enzyme, e.g., PNGaseF, and (ii) separating the N-linked glycans in a mobile phase of a mixed mode chromatography. In some aspects, the disclosure provides a method of quantifying a glycosylation profile of a recombinant protein comprising (i) releasing one or more N-linked glycans using an enzyme, e.g., PNGaseF, (ii) separating the N-linked glycans in a mobile phase of a mixed mode chromatography, and (iii) measuring the separated N-linked glycans using a detector. The method of the invention comprises: (i) releasing one or more N-linked glycans from the recombinant protein by an enzyme prior to analysis, (ii) separating the released N-linked glycans using a chromatography column, and (iii) measuring the separated N-linked glycans by ELSD, NQAD, refractive index detector, or a charged aerosol detector (CAD).

In some aspects, the enzyme is incubated with the recombinant protein, wherein the one or more N-linked glycans are released from the recombinant protein prior to the separation. In some aspects, the enzyme is diluted in buffer. In some aspects, the buffer is selected from a group consisting of phosphate, citrate, formate, acetate, and Tris (hydroxymethyl)-aminomethane ("Tris"). In some aspects, the buffer is phosphate. In some aspects, the buffer is citrate. In some aspects, the buffer is formate. In some aspects, the buffer is acetate. In some aspects, the buffer is Tris.

In an aspect described herein, the methods analyze the N-linked glycans via a mass spectrometry. One common mass spectrometry approach is to use three mass spectrometers in tandem, known as mass spectrometry/mass spectrometry (MS/MS). For example, in an experiment on a triple-quadrupole mass spectrometer, the first quadrupole (Q1) is set to pass ions only of a specified m/z (precursor ions) of an expected chemical species in the sample. The second quadrupole (i.e. Q2 or the collision cell) is used to fragment the ions passing through Q1. The third quadrupole (Q3) is set to pass to the detector only ions of a specified m/z (fragment ions) corresponding to an expected fragmentation product of the expected chemical species. One example of a mass spectrometer that can be used in the current process is an NQAD, which is a device used to monitor the mass/charge ratio of components that pass through the detector as described above. In some aspects, the one or more N-linked glycans that are separated are measured by a mass spectrometer. In some aspects, the one or more N-linked glycans that are separated are characterized by a mass spectrometer. In some aspects, the one or more N-linked glycans that are separated are detected by a mass spectrometer.

The methods of the present disclosure are also useful to analyze N-linked glycans via an evaporative light scattering detector, or ELSD. An evaporative light scattering detector (ELSD) is a detector used in conjunction with high-performance liquid chromatography (HPLC), Ultra high-performance liquid chromatography (UHPLC). ELSDs analyze solvent after elution from HPLC. As the eluent passes from an HPLC, it is mixed with an inert carrier gas and forced through a nebulizer, which separates the liquid into minute aerosolized droplets. This spray is then heated so that only the mobile phase evaporates, and light is focused on the remaining target substance, and scattered light is detected. ELSD detectors are useful to identify samples that cannot be identified via other common methods such as Ultraviolet (UV) detection because components do not absorb UV light. In some aspects, the one or more N-linked glycans that are separated are measured by an evaporative light scattering detector. In some aspects, the one or more N-linked glycans that are separated are characterized by an evaporative light scattering detector. In some aspects, the one or more N-linked glycans that are separated are detected by an evaporative light scattering detector.

The methods of the present disclosure also are useful to analyze N-linked glycans using a refractive index detector. A refractive index detector is a device used in conjunction with high-performance liquid chromatography (HPLC), Ultra high-performance liquid chromatography (UHPLC). The device operates on a detection principle involving measuring the change in refractive index of a column eluate passing through, and is capable of detecting differences in the refractive index of a sample and a mobile phase. Refractive index detectors can contain a flow cell with two parts: one for the sample and one for the reference solvent. The detector measures the refractive index of both components and in effect, subtracts the mobile-phase background signal from the sample signal. In some aspects, the one or more N-linked glycans that are separated are measured by a refractive index detector. In some aspects, the one or more N-linked glycans that are separated are characterized by a refractive index detector. In some aspects, the one or more N-linked glycans that are separated are detected by a refractive index detector.

The methods of the present disclosure are also useful to analyze N-linked glycans using a charged aerosol detector. Charged aerosol detection refers to an analytical technique based on the nebulization of an eluent with a nitrogen (or air) carrier gas to form droplets that are then dried to remove mobile phase, producing analyte particles. The primary stream of analyte particles is met by a secondary stream that is positively charged as a result of having passed a high-voltage, platinum corona wire. The charge transfers difusionally to the opposing stream of analyte particles, and is further transferred to a collector where it is measured by a highly sensitive electrometer, generating a signal in direct proportion to the quantity of analyte present. In some aspects, the one or more N-linked glycans that are separated are measured by a charged aerosol detector (CAD). In other aspects, the one or more N-linked glycans that are separated are measured by ELSD, NQAD, or refractive index detector.

The methods of the present disclosure involve separation of the released glycans via chromatography, e.g., liquid chromatography (LC). LC is a well-established analytical technique for separating components of a fluidic mixture for subsequent analysis and/or identification, in which a column, microfluidic chip-based channel, or tube is packed with a stationary phase material that typically is a finely divided solid or gel such as small particles with diameter of a few microns. The small particle size provides a large surface area that can be modified with various chemistries creating a stationary phase. A liquid eluent is pumped through the liquid chromatographic column ("LC column") at a desired flow rate based on the column dimensions and particle size. This liquid eluent is sometimes referred to as the mobile phase. The sample to be analyzed is introduced (e.g., injected) in a small volume into the stream of the mobile phase prior to the LC column. The migration rates of analytes in the sample are affected by specific chemical and/or physical interactions with the stationary phase as they traverse the length of the column. The time at which a specific analyte elutes or comes out of the end of the column is called the retention time or elution time and can be a reasonably identifying characteristic of a given analyte.

In some aspects, the chromatography to separate the N-linked glycans for the present methods comprises one or more mobile phases. In some aspects, the chromatography comprises at least two mobile phases, at least three mobile phases, at least four mobile phases, or at least five mobile phases. In other aspects, the chromatography comprises a first mobile phase and a second mobile phase. In other aspects, the chromatography comprises a first mobile phase, a second mobile phase, or a third mobile phase. In other aspects, the chromatography comprises a first mobile phase, a second mobile phase, a third mobile phase, or a fourth mobile phase. In other aspects, the chromatography comprises a first mobile phase, a second mobile phase, a third mobile phase, a fourth mobile phase, or a fifth mobile phase. In some aspects, the first mobile phase, the second mobile phase, the third mobile phase, the fourth mobile phase, and/or the fifth mobile phase are different.

In some aspects, the first mobile phase, the second mobile phase, the third mobile phase, the fourth mobile phase, or the fifth mobile phase is selected from a group consisting of hexane, methanol, water, acetonitrile, ethyl acetate, benzene, chloroform, benzene, ether, or a mixture thereof. In some aspects, the first mobile phase, the second mobile phase, the third mobile phase, the fourth mobile phase, or the fifth mobile phase is water. In some aspects, the first mobile phase, the second mobile phase, the third mobile phase, the fourth mobile phase, or the fifth mobile phase is acetonitrile. In some aspects, the first mobile phase, the second mobile phase, the third mobile phase, the fourth mobile phase, or the fifth mobile phase is ethyl acetate. In some aspects, the first mobile phase, the second mobile phase, the third mobile phase, the fourth mobile phase, or the fifth mobile phase is benzene. In some aspects, the first mobile phase, the second mobile phase, the third mobile phase, the fourth mobile phase, or the fifth mobile phase is chloroform. In some aspects, the first mobile phase, the second mobile phase, the third mobile phase, the fourth mobile phase, or the fifth mobile phase is benzene. In some aspects, the first mobile phase, the second mobile phase, the third mobile phase, the fourth mobile phase, or the fifth mobile phase is ether.

In some aspects, the first mobile phase comprises water. In some aspects, the first mobile phase comprises water and the second mobile phase comprises acetonitrile. In some aspects, the first mobile phase is water and further comprises formic acid (FA) and the second phase is acetonitrile. In some aspects, the first mobile phase is water and the second phase is acetonitrile and further comprises formic acid (FA). In some aspects, the first mobile phase is water and further comprises formic acid (FA) and the second phase is acetonitrile and further comprises formic acid (FA). In some aspects, the first mobile phase is water and further comprises from about 0.01% to about 1% formic acid (FA) and the second phase is acetonitrile. In some aspects, the first mobile phase is water and the second phase is acetonitrile and further comprises from about 0.01% to about 1% formic acid (FA). In some aspects, the first mobile phase is water and further comprises from about 0.01% to about 1% formic acid (FA) and the second phase is acetonitrile and further comprises from about 0.01% to about 1% formic acid (FA). In some aspects, the first mobile phase and the second mobile phase comprise about 0.01% formic acid (FA). In some aspects, the first mobile phase and the second mobile phase comprise about 0.02% formic acid (FA). In some aspects, the first mobile phase and the second mobile phase comprise about 0.03% formic acid (FA). In some aspects, the first mobile phase and the second mobile phase comprise about 0.04% formic acid (FA). In some aspects, the first mobile phase and the second mobile phase comprise about 0.05% formic acid (FA). In some aspects, the first mobile phase and the second mobile phase comprise about 0.06% formic acid (FA). In some aspects, the first mobile phase and the second mobile phase comprise about 0.07 formic acid (FA). In some aspects, the first mobile phase and the second mobile phase comprise about 0.08% formic acid (FA). In some aspects, the first mobile phase and the second mobile phase comprise about 0.09% formic acid (FA). In some aspects, the first mobile phase and the second mobile phase comprise about 0.1% formic acid (FA).

In some aspects, the first mobile phase comprises water. In some aspects, the first mobile phase comprises water and the second mobile phase comprises acetonitrile. In some aspects, the first mobile phase is water and further comprises trifluoroacetic acid (TFA) and the second phase is acetonitrile. In some aspects, the first mobile phase is water and the second phase is acetonitrile and further comprises trifluoroacetic acid (TFA). In some aspects, the first mobile phase is water and further comprises trifluoroacetic acid (TFA) and the second phase is acetonitrile and further comprises trifluoroacetic acid (TFA). In some aspects, the first mobile phase is water and further comprises from about 0.01% to about 1% trifluoroacetic acid (TFA) and the second phase is acetonitrile. In some aspects, the first mobile phase is water and the second phase is acetonitrile and further comprises from about 0.01% to about 1% trifluoroacetic acid (TFA). In some aspects, the first mobile phase is water and further comprises from about 0.01% to about 1% trifluoroacetic acid (TFA) and the second phase is acetonitrile and further comprises from about 0.01% to about 1% trifluoroacetic acid (TFA). In some aspects, the first mobile phase and the second mobile phase comprise about 0.01% trifluoroacetic acid (TFA). In some aspects, the first mobile phase and the second mobile phase comprise about 0.02% trifluoroacetic acid (TFA). In some aspects, the first mobile phase and the second mobile phase comprise about 0.03% trifluoroacetic acid (TFA). In some aspects, the first mobile phase and the second mobile phase comprise about 0.04% trifluoroacetic acid (TFA). In some aspects, the first mobile phase and the second mobile phase comprise about 0.05% trifluoroacetic acid (TFA). In some aspects, the first mobile phase and the second mobile phase comprise about 0.06% trifluoroacetic acid (TFA). In some aspects, the first mobile phase and the second mobile phase comprise about 0.07 trifluoroacetic acid (TFA). In some aspects, the first mobile phase and the second mobile phase comprise about 0.08% trifluoroacetic acid (TFA). In some aspects, the first mobile phase and the second mobile phase comprise about 0.09% trifluoroacetic acid (TFA). In some aspects, the first mobile phase and the second mobile phase comprise about 0.1% trifluoroacetic acid (TFA).

In some aspects, the first mobile phase comprises water. In some aspects, the first mobile phase comprises water and the second mobile phase comprises acetonitrile. In some aspects, the first mobile phase is water and further comprises triethylamine (TEA) and the second phase is acetonitrile. In some aspects, the first mobile phase is water and the second phase is acetonitrile and further comprises triethylamine (TEA). In some aspects, the first mobile phase is water and further comprises triethylamine (TEA) and the second phase is acetonitrile and further comprises triethylamine (TEA). In some aspects, the first mobile phase is water and further comprises from about 0.01% to about 1% triethylamine (TEA) and the second phase is acetonitrile. In some aspects, the first mobile phase is water and the second phase is acetonitrile and further comprises from about 0.01% to about 1% triethylamine (TEA). In some aspects, the first mobile phase is water and further comprises from about 0.01% to about 1% triethylamine (TEA) and the second phase is acetonitrile and further comprises from about 0.01% to about 1% triethylamine (TEA). In some aspects, the first mobile phase and the second mobile phase comprise about 0.01% triethylamine (TEA). In some aspects, the first mobile phase and the second mobile phase comprise about 0.02% triethylamine (TEA). In some aspects, the first mobile phase and the second mobile phase comprise about 0.03% triethylamine (TEA). In some aspects, the first mobile phase and the second mobile phase comprise about 0.04% triethylamine (TEA). In some aspects, the first mobile phase and the second mobile phase comprise about 0.05% triethylamine (TEA). In some aspects, the first mobile phase and the second mobile phase comprise about 0.06% triethylamine (TEA). In some aspects, the first mobile phase and the second mobile phase comprise about 0.07% triethylamine (TEA). In some aspects, the first mobile phase and the second mobile phase comprise about 0.08% triethylamine (TEA). In some aspects, the first mobile phase and the second mobile phase comprise about 0.09% triethylamine (TEA). In some aspects, the first mobile phase and the second mobile phase comprise about 0.1% triethylamine (TEA).

In some aspects, the first mobile phase is water and further comprises triethylamine (TEA) and the second phase is acetonitrile. In some aspects, the first mobile phase is water and further comprises triethylamine (TEA) and the second phase is acetonitrile and further comprises triethylamine (TEA). In some aspects, the first mobile phase is water and further comprises from about 0.05% to about 1.5% triethylamine (TEA) and the second phase is acetonitrile, wherein glycans S1G2, S2G2, S2G2F, in any combination, have increased affinity for the chromatography column during separation compared to 0.1% formic acid in mobile phase A and/or mobile phase B. In some aspects, the first mobile phase is water and further comprise triethylamine (TEA) and the second phase is acetonitrile and further comprises from about 0.05% to about 1.5% triethylamine (TEA), wherein glycans S1G2, S2G2, S2G2F, in any combination, have increased affinity for the chromatography column during separation compared to 0.1% formic acid in mobile phase A and/or mobile phase B. In some aspects, the first mobile phase is water and further comprises from about 0.05% to about 1.5% triethylamine (TEA) and the second phase is acetonitrile and further comprises from about 0.05% to about 1.5% triethylamine (TEA), wherein glycans S1G2, S2G2, S2G2F, in any combination, have increased affinity for the chromatography column during separation compared to 0.1% formic acid in mobile phase A and/or mobile phase B. In some aspects, the first mobile phase comprises about 0.1% trimethylamine (TEA) in water and the second mobile phase comprise about 0.1% triethylamine (TEA) in acetonitrile. In some aspects, the first mobile phase comprises about 0.05% triethylamine (TEA). In some aspects, the first mobile phase comprises about 0.06% triethylamine (TEA). In some aspects, the first mobile phase comprises about 0.07% triethylamine (TEA). In some aspects, the first mobile phase comprises about 0.08% triethylamine (TEA). In some aspects, the first mobile phase comprises about 0.09% triethylamine (TEA). In some aspects, the first mobile phase comprises about 0.1% triethylamine (TEA). In some aspects, the first mobile phase comprises about 0.11% triethylamine (TEA). In some aspects, the first mobile phase comprises about 0.12% triethylamine (TEA). In some aspects, the first mobile phase comprises about 0.13% triethylamine (TEA). In some aspects, the first mobile phase comprises about 0.14% triethylamine (TEA). In some aspects, the first mobile phase comprises about 0.15% triethylamine (TEA). In some aspects, the second mobile phase comprises about 0.05% triethylamine (TEA). In some aspects, the second mobile phase comprises about 0.06% triethylamine (TEA). In some aspects, the second mobile phase comprises about 0.07% triethylamine (TEA). In some aspects, the second mobile phase comprises about 0.08% triethylamine (TEA). In some aspects, the second mobile phase comprises about 0.09% triethylamine (TEA). In some aspects, the second mobile phase comprises about 0.10% triethylamine (TEA). In some aspects, the second mobile phase comprises about 0.11% triethylamine (TEA). In some aspects, the second mobile phase comprises about 0.12% triethylamine (TEA). In some aspects, the second mobile phase comprises about 0.13% triethylamine (TEA). In some aspects, the second mobile phase comprises about 0.14% triethylamine (TEA). In some aspects, the second mobile phase comprises about 0.15% triethylamine (TEA).

In some aspects, the first mobile phase, the second mobile phase, the third mobile phase, the fourth mobile phase, or the fifth mobile phase comprise formic acid (FA), Trifluoroacetic acid (TFA), Triethylamine (TEA), or any combination thereof. In some aspects, the first mobile phase, the second mobile phase, the third mobile phase, the fourth mobile phase, or the fifth mobile phase comprise from about 0.01% to about 2% formic acid (FA), Trifluoroacetic acid (TFA), Triethylamine (TEA), or any combination thereof.

In some aspects, the first mobile phase, the second mobile phase, the third mobile phase, the fourth mobile phase, or the fifth mobile phase comprise from about 0.01% to about 1% or from about 1% to about 2% formic acid (FA), e.g., 0.1% FA. In some aspects, the first mobile phase, the second mobile phase, the third mobile phase, the fourth mobile phase, or the fifth mobile phase comprise from about 0.01% to about 1% or from about 1% to about 2% Trifluoroacetic acid (TFA), e.g., 0.1% TFA. In some aspects, the first mobile phase, the second mobile phase, the third mobile phase, the fourth mobile phase, or the fifth mobile phase comprise from about 0.01% to about 1% or from about 1% to about 2% Triethylamine (TEA), e.g., 0.1% TEA, wherein glycans S1G2, S2G2, and/or S2G2F have increased affinity for the chromatography column during separation compared to 0.1% formic acid in mobile phase A and/or mobile phase B.

In some aspects, the methods of the present disclosure use one or more agents for reductive amination to improve peak symmetry and reduce peak tailing during chromatographic separations. In some aspects, the one or more agents comprise 2-picoline borane (pic-BH₃) and/or sodium cyanoborohydride (NaBH₃CN).

The methods of the present disclosure are useful to separate N-glycans using chromatography. The chromatography separations can be improved by adjusting the temperature of the chromatography column during separation. In some aspects, the separation is performed at a temperature lower than 70 °C. In some aspects, the separation is performed at a temperature lower than 60 °C. In some aspects, the separation is performed at a temperature lower than 50 °C. In some aspects, the separation is performed at a temperature lower than 40 °C.

In some aspects, the temperature is between about 50 °C and about 70 °C, about 50 °C and about 60 °C, about 60 °C and about 70 °C, about 55 °C and about 65 °C, about 55 °C and about 60 °C, about 60 °C and about 65 °C, about 65 °C and about 70 °C, about 50 °C and about 55 °C, about 50 °C and about 60 °C, about 50 °C and about 59 °C, about 51 °C and about 59 °C, about 51 °C and about 58 °C, about 52 °C and about 58 °C, about 52 °C and about 57 °C, about 53 °C and about 57 °C, about 53 °C and about 56 °C, and about 54 °C and about 56 °C.

In some aspects, the temperature is between about 50 °C and about 70 °C. In some aspects, the temperature is between about 50 °C and about 60 °C. In some aspects, the temperature is between about 60 °C and about 70 °C. In some aspects, the temperature is between about 55 °C and about 65 °C. In some aspects, the temperature is between about 55 °C and about 60 °C. In some aspects, the temperature is between about 60 °C and about 65 °C. In some aspects, the temperature is between about 65 °C and about 70 °C. In some aspects, the temperature is between about 50 °C and about 55 °C. In some aspects, the temperature is between about 50 °C and about 60 °C. In some aspects, the temperature is between about 50 °C and about 59 °C. In some aspects, the temperature is between about 51 °C and about 59 °C. In some aspects, the temperature is between about 51 °C and about 58 °C. In some aspects, the temperature is between about 52 °C and about 58 °C. In some aspects, the temperature is between about 52 °C and about 57 °C. In some aspects, the temperature is between about 53 °C and about 57 °C. In some aspects, the temperature is between about 53 °C and about 56 °C. In some aspects, the temperature is between and about 54 °C and about 56 °C.

In some aspects, the temperature is between about 45 °C and about 55 °C, about 45 °C and about 54 °C, about 46 °C and about 54 °C, about 46 °C and about 53 °C, about 47 °C and about 53 °C, about 47 °C and about 52 °C, about 48 °C and about 52 °C, about 49 °C and about 52 °C, or about 49 °C and about 51 °C. In some aspects, the temperature is between about 55 °C and about 65 °C, about 55 °C and about 64 °C, about 56 °C and about 64 °C, about 56 °C and about 63 °C, about 57 °C and about 63 °C, about 57 °C and about 62 °C, about 58 °C and about 62 °C, about 59 °C and about 62 °C, or about 59 °C and about 61 °C.

In some aspects, the temperature is about 50 °C, about 51 °C, about 52 °C, about 53 °C, about 54 °C, about 55 °C, about 56 °C, about 57 °C, about 58 °C, about 59 °C, about 60 °C, about 61 °C, about 62 °C, about 63 °C, about 64 °C, about 65 °C, about 66 °C, about 67 °C, about 68 °C, or about 69 °C.

In some aspects, the temperature is between about 45 °C and about 55 °C. In some aspects, the temperature is between about 45 °C and about 54 °C. In some aspects, the temperature is between about 46 °C and about 54 °C. In some aspects, the temperature is between about 46 °C and about 53 °C. In some aspects, the temperature is between about 47 °C and about 53 °C. In some aspects, the temperature is between about 47 °C and about 52 °C. In some aspects, the temperature is between about 48 °C and about 52 °C. In some aspects, the temperature is between about 49 °C and about 52 °C. In some aspects, the temperature is between about 49 °C and about 51 °C. In some aspects, the temperature is between about 55 °C and about 65 °C. In some aspects, the temperature is between about 55 °C and about 64 °C. In some aspects, the temperature is between about 56 °C and about 64 °C. In some aspects, the temperature is between about 56 °C and about 63 °C. In some aspects, the temperature is between about 57 °C and about 63 °C. In some aspects, the temperature is between about 57 °C and about 62 °C. In some aspects, the temperature is between about 58 °C and about 62 °C. In some aspects, the temperature is between about 59 °C and about 62 °C. In some aspects, the temperature is between about 59 °C and about 61 °C.

The methods of the present disclosure are useful to separate N-linked glycans using chromatography. Gradients in reversed-phase HPLC usually involve the on-line (dynamic) mixing of solvents (mobile phases) to achieve a steady increase in one solvent over the course of the separation, which serves to alter the elution strength of the eluent over time. In some aspects, the separation is on a gradient. In some aspects, the separation is on a gradient of one or more mobile phases. In some aspects, the gradient is from the first mobile phase to the second mobile phase. In some aspects, the gradient is from the first mobile phase to the second mobile phase to the third mobile phase. In some aspects, the gradient is from the first mobile phase to the second mobile phase to the third mobile phase to the fourth mobile phase. In some aspects, the gradient is from the first mobile phase to the second mobile phase to the third mobile phase to the fourth mobile phase to the fifth mobile phase. In some aspects, the gradient is from about 95% to about 5%. In some aspects, the gradient is from about 95% to about 50%, from about 95% to about 55%, from about 95% to about 60%, from about 95% to about 65%, from about 95% to about 70%, from about 95% to about 75%, from about 95% to about 80%, from about 95% to about 85%, from about 90% to about 50%, from about 90% to about 55%, from about 90% to about 60%, from about 90% to about 65%, from about 90% to about 70%, from about 90% to about 75%, from about 87% to about 50%, from about 87% to about 55%, from about 87% to about 60%, from about 87% to about 65%, from about 87% to about 70%, from about 87% to about 75%, from about 85% to about 50%, from about 85% to about 55%, from about 85% to about 60%, from about 85% to about 65%, from about 85% to about 70%, or from about 85% to about 75%. In some aspects, the gradient is from about 87% to about 75%.

In some aspects, the gradient is from about 95% to about 50%. In some aspects, the gradient is from about 95% to about 51%. In some aspects, the gradient is from about 95% to about 52%. In some aspects, the gradient is from about 95% to about 53%. In some aspects, the gradient is from about 95% to about 54%. In some aspects, the gradient is from about 95% to about 55%. In some aspects, the gradient is from about 95% to about 56%. In some aspects, the gradient is from about 95% to about 57%. In some aspects, the gradient is from about 95% to about 58%. In some aspects, the gradient is from about 95% to about 59%. In some aspects, the gradient is from about 95% to about 60%. In some aspects, the gradient is from about 90% to about 74%. In some aspects, the gradient is from about 90% to about 75%. In some aspects, the gradient is from about 90% to about 76%. In some aspects, the gradient is from about 90% to about 77%. In some aspects, the gradient is from about 90% to about 78%. In some aspects, the gradient is from about 90% to about 79%. In some aspects, the gradient is from about 90% to about 80%. In some aspects, the gradient is from about 90% to about 81%. In some aspects, the gradient is from about 90% to about 82%. In some aspects, the gradient is from about 90% to about 83%. In some aspects, the gradient is from about 90% to about 84%. In some aspects, the gradient is from about 90% to about 85%. In some aspects, the gradient is from about 87% to about 50%. In some aspects, the gradient is from about 87% to about 51%. In some aspects, the gradient is from about 87% to about 52%. In some aspects, the gradient is from about 87% to about 53%. In some aspects, the gradient is from about 87% to about 54%. In some aspects, the gradient is from about 87% to about 55%. In some aspects, the gradient is from about 87% to about 56%. In some aspects, the gradient is from about 87% to about 57%. In some aspects, the gradient is from about 87% to about 58%. In some aspects, the gradient is from about 87% to about 59%. In some aspects, the gradient is from about 87% to about 60%. In some aspects, the gradient is from about 87% to about 61%. In some aspects, the gradient is from about 87% to about 62%. In some aspects, the gradient is from about 87% to about 63%. In some aspects, the gradient is from about 87% to about 64%. In some aspects, the gradient is from about 87% to about 65%. In some aspects, the gradient is from about 87% to about 66%. In some aspects, the gradient is from about 87% to about 67%. In some aspects, the gradient is from about 87% to about 68%. In some aspects, the gradient is from about 87% to about 69%. In some aspects, the gradient is from about 87% to about 70%. In some aspects, the gradient is from about 87% to about 71%. In some aspects, the gradient is from about 87% to about 72%. In some aspects, the gradient is from about 87% to about 73%. In some aspects, the gradient is from about 87% to about 74%. In some aspects, the gradient is from about 87% to about 75%.

### III. Methods of Glycan Analysis

The methods of the present disclosure are also useful for analyzing released and separated N-linked glycans as shown above. N-linked glycosylation is the attachment of an oligosaccharide, sometimes also referred to as glycan, to a nitrogen atom (the amide nitrogen of an asparagine (Asn) residue of a protein), in a process called N-glycosylation. The present disclosure is also directed to a method of analyzing the N-linked glycan profile of a protein of interest. In some aspects, the one or more N-linked glycans are Galactose (Gal), N-Acetylgalactosamine (GalNAc), Galactosamine (GalN), Glucose (Glc), N-Acetylglucosamine (GlcNAc), Glucosamine (GlcN), Mannose (Man), N-Acetylmannosamine (ManNAc), Mannosamine (ManN), Xylose (Xyl), Fucose (Fuc), Glucuronic Acid (GlcA), Iduronic acid (IdoA), Galacturonic acid (GalA), Mannuronic acid (ManA), or any combination thereof. In some aspects, the N-glycan is Gal. In some aspects, the N-glycan is GalNac. In some aspects, the N-glycan is GalN. In some aspects, the N-glycan is Glc. In some aspects, the N-glycan is GlcNAc. In some aspects, the N-glycan is GlcN. In some aspects, the N-glycan is Man. In some aspects, the N-glycan is ManNAc. In some aspects, the N-glycan is ManN. In some aspects, the N-glycan is Xyl. In some aspects, the N-glycan is Fuc. In some aspects, the N-glycan is GlcA. In some aspects, the N-glycan is IdoA. In some aspects, the N-glycan is GalA. In some aspects, the N-glycan is ManA. In some aspects, the one or more N-glycans comprise one or more bi-antennary glycans. In some aspects, the bi-antennary glycans are selected from a group consisting of G0F, G0, G1F, G1, G2F, G2, S1G2F, S1G2, S2G2F, S2G2 and any combination thereof. In some aspects, the bi-antennary glycan is G0F or G0. In some aspects, the bi-antennary glycan is G1F or G1. In some aspects, the bi-antennary glycan is G2F or G2. In some aspects, the bi-antennary glycan is S1G2F or S1G2. In some aspects, the bi-antennary glycan is S2G2F or S2G2.

In some aspects, the one or more N-linked glycans are high mannose glycans. High-mannose glycans contain unsubstituted terminal mannose sugars. These glycans typically contain between five and nine mannose residues attached to the chitobiose (GlcNAc₂) core. In some aspects, the one or more high mannose N-linked glycans is Mannose-5. In some aspects, the one or more high mannose N-linked glycans is Mannose-6. In some aspects, the one or more high mannose N-linked glycans is Mannose-7. In some aspects, the one or more high mannose N-linked glycans is Mannose-8. In some aspects, the one or more high mannose N-linked glycans is Mannose-9.

The high mannose N-linked glycans can also be phosphorylated. Mannose-6-phosphate (M6P) is a crucial signal for the trafficking of lysosomal enzymes to lysosomes. Most lysosomal enzymes are glycoproteins with high-mannose type glycans as well as complex type glycans. For example, FABRAZYME^{™} is one example of a lysosomal protein, α-Galactosidase A, wherein the ability of FABRAZYME^{™} to be taken up by cells and subsequently be transported to the lysosome is due to the presence of mannose 6-phosphate (M6P) on its N-linked glycans, wherein FABRAZYME^{™} is delivered to lysosomes through binding to the mannose-6-phosphate/IGF-II receptor present on the cell surface of most cell types. In some aspects, one or more mannose residues in the high mannose N-linked glycans are phosphorylated.

The methods of the present disclosure are also useful for separating glycans comprising sialyation. Sialylation is the process by which sialic acid groups are introduced onto molecules such as oligosaccharides and carbohydrates as the terminal monosaccharide. There are two common mammalian sialic acids: N-acetylneuraminic acid (Neu5Ac or NANA) and N-glycolylneuraminic acid (Neu5Gc or NGNA). In some aspects, the sialic acid is Neu5Ac. In some aspects, the sialic acid is Neu5Gc. In some aspects, the sialic acid is 2-Keto-3-deoxynononic acid (Kdn). In some aspects, the glycosylation profile comprises one or more asialylated glycans, mono-sialylated glycans, di-sialylated glycans, and/or tri-sialylated and tetra-sialylated glycans. In some aspects, the glycosylation profile comprises one or more asialylated glycans. In some aspects, the glycosylation profile comprises one or more mono-sialylated glycans. In some aspects, the glycosylation profile comprises one or more di-sialylated glycans. In some aspects, the glycosylation profile comprises one or more tri-sialylated glycans. In some aspects, the glycosylation profile comprises one or more tetra-sialylated glycans.

The methods of the present disclosure are useful for characterizing the glycosylation profiles of proteins. In some aspects, the proteins are recombinant proteins. In some aspects, the recombinant protein is at least about 80%, at least about 85%, at least about 90%, at least about 95%, at least about 96%, at least about 97%, at least about 98%, at least about 99%, or about 100% pure.

In some aspects, the recombinant protein is a fusion protein. In some aspects, the fusion protein is an anti-Myostatin fusion protein. In some aspects, the fusion protein is talditercept alfa. In other aspects, the fusion protein is an Fc fusion protein. In some aspects, the fusion protein is a homodimer of an Fc fusion protein. In some aspects, a fusion protein comprises a CTLA-4 extracellular fusion protein. In some aspects, a CTLA-4 Fc fusion protein is abatacept. In other aspects, a CTLA-4 Fc fusion protein is belatacept. CTLA4-Ig molecules, uses and methods thereof, are also described in U.S. Patent No. 5,434,131; 5,851,795; 5,885,796; 5,885,579; and 7,094,874. In some aspects, the CTLA-4-Fc fusion protein comprises at least one N-linked glycan, at least two N-linked glycans, or at least three N-liked glycans, e.g., T5, T7, and T15. As used herein, "T5", T7", and T15" refer to specific glycosylation sites present on the abatacept or belatacept molecule. These labels correspond to Asparagine 76, Asparagine 108, and Asparagine 207, respectively, and correspond to the residues in SEQ ID NO: 3 and SEQ ID NO: 5 (bolded).
SEQ ID NO: 1 [CTLA4 extracellular domain sequence]
Abatacept (SEQ ID NO:3) [amino acids 27-383 of SEQ ID NO: 2]
Belatacept (SEQ ID NO: 5) [amino acids 27-383 of SEQ ID NO: 4]

MHVAQPAVVLASSRGIASFVCEYASPGKYTEVRVTVLRQADSQVTEVCAATYMMGNELTFLDDSICTG
TSSGNQVNLTIQGLRAMDTGLYICKVELMYPPPYYEGIGNGTQIYVIDPEPCPDSDQEPKSSDKTHTS
PPSPAPELLGGSSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHE
ALHNHYTQKSLSLSPGK

In some aspects, the recombinant protein is an antibody. In some aspects, the antibody is an isotype selected from IgM, IgA, IgE, IgD, and IgG. In some aspects, the antibody is isotype IgM. In some aspects, the antibody is isotype IgA. In some aspects, the antibody is isotype IgE. In some aspects, the antibody is isotype IgD. In some aspects, the antibody is isotype IgG. In some aspects, the IgG antibody is selected from IgG1, IgG2, IgG3, and IgG4. In some aspects, the antibody is bispecific. In some aspects, the antibody is multispecific.

The methods of the present disclosure are useful to analyze antibodies that are useful for therapeutic purposes. In some aspects, the antibody is an anti-GITR antibody, an anti-CXCR4 antibody, an anti-CD73 antibody, an anti-TIGIT antibody, an anti-OX40 antibody, an anti-LAG3 antibody, an anti-CSF1R antibody, or an anti-IL8 antibody. In some aspects, the antibody has a single N-linked glycosylation site. In some aspects, the single N-linked glycosylation site is Asparagine 297 (N297). In some aspects, the antibody has at least one N-linked glycosylation site, at least two N-linked glycosylation sites, at least three N-linked glycosylation sites, at least four N-linked glycosylation sites, or at least five N-linked glycosylation sites.

The methods of the present disclosure are also useful to analyze the N-linked glycans of other recombinant proteins. In some aspects, the recombinant protein comprises an enzyme, a hormone, a cytokine, a cell surface receptor, a protease, a cytokine receptor, or any combination thereof. In some aspects, the recombinant protein is a fusion protein. In some aspects, the fusion protein is fused to a heterologous moiety. In some aspects, the heterologous moiety is a half-life extending moiety. In some aspects, the half-life extending moiety comprises albumin, albumin binding polypeptide, a fatty acid, PAS, the β subunit of the C-terminal peptide (CTP) of human chorionic gonadotropin, polyethylene glycol (PEG), hydroxyethyl starch (HES), XTEN, albumin-binding small molecules, Fc, or a combination thereof. In some aspects, the half-life extending moiety is an Fc.

In some aspects, the present methods are useful to analyze the N-linked glycans of a fusion protein, e.g., an anti-PD-L1/TGFβR2 bispecific fusion protein. See Jochems et al., Oncotarget. 2017 Sep 26; 8(43): 75217-75231.

In some aspects, the present methods are useful to analyze BsAb-HAS (human serum albumin) fusion proteins. Various BsAbs includes, but are not limited to, BsAb fragments such as scFv-HSA-scFv, scDiabody-HSA, tandem scFv-HSA, etc.

In some aspects, the present methods are useful to analyze BsAb-Toxin fusion proteins. BsAb-toxin fusion proteins are the extension of immunotoxin which consists of a protein toxin, working as the cytotoxic portion, fused to the portion of an antibody. Among which, tandem scFv-toxin is the most reported format, also known as bispecific ligand-directed toxin (BLT). The catalytic toxins, such as pseudomonas exotoxin (PE) and diphtheria toxin (DT) are usually selected as the cytotoxic portion.

The methods of the present disclosure are also useful for biomanufacturing analysis and batch release analysis. Batch release testing is generally required by Good Manufacturing Practice (GMP) and a necessary requirement to ensure high quality pharmaceuticals and biopharmaceuticals prior to release for sale, supply or export. In some aspects, the method is a batch release assay.

The present disclosure is further illustrated by the following examples.

### EXAMPLES

### EXAMPLE 1

### Reagents, Columns and Systems

Recombinant PNGase F and Rapid PNGase F were purchased from New England Biolabs, Inc. (Ipswich, MA). Glycan standards were purchased from Prozyme (Hayward, CA). Water and Acetonitrile for mobile phases were purchased from J.T. Baker via Fisher Scientific (Hampton, NH). All mobile phase additives (formic acid, trifluoro acetic acid, triethylamine, etc.) were purchased from Sigma Aldrich (St. Louis, MO). AdvanceBio N-Glycan Cleanup Cartridges were purchased from Agilent Technologies (Santa Clara, CA).

A variety of columns were screened during development, which included HILIC, Reversed Phase and Porous Graphite Carbon separations. For HILIC separation, XBridge Glycan BEH Amide^{™} 130 Å, 2.5 µm (2.1 x 150 mm) and Acquity UPLC Glycan BEH Amide^{™} 130 Å, 1.7 µm (2.1 x 150 mm) columns were purchased from Waters Corporation (Milford, MA). Additionally, PolyGLYCOPLEX^{™} A 3 µm (2.1 x 100 mm) column was purchased from PolyLC Inc. (Columbia, MD), and the TSKGel Amide-80 2 µm (2.0 x 150 mm) column was purchased from Tosoh Biosciences LLC (Tokyo, Japan). For evaluation of reversed phase, the XBridge BEH C18 2.5 µm (3.0 x 150 mm) column, Acquity UPLC Peptide CSH C18 130 Å, 1.7 µm (2.1 x 100 mm) column and CORTECS UPLC C18+^{™}, 1.6 µm (2.1 x 100 mm) column were all purchased from Waters Corporation. The HYPERCARB^{™} 3 µm (2.1 x 100 mm) column was purchased from Thermo Scientific^{™} (Waltham, MA). All chromatographic separations were performed on an Alliance 2695 HPLC^{™} system or an Acquity H-Class UPLC^{™} system connected to a CORONA^{™} VEO^{™} Charged Aerosol Detector system from Thermo Scientific^{™} for detection of glycans.

### Optimized Release of Oligosaccharides

Oligosaccharides were released from mAb samples by diluting 1 mg of mAb with 10 µL Rapid PNGase F Buffer and 20 mM Tris Buffer at pH 7.5 with a final volume of 180 µL. The samples were then vortex mixed briefly and centrifuged to collect supernatant. Rapid PNGase F was diluted 1:10 with HPLC water and 20 µL of this solution was added to each reaction vial, vortexed and centrifuged. The Rapid PNGase F solution was incubated for 60 minutes at 50 °C and cooled to room temperature. In order to remove protein and buffer components, the oligosaccharides were extracted using Agilent AdvanceBio N-Glycan Deglycosylation Cleanup Cartridges^{™}. The cartridges were first equilibrated with 500 µL water twice and then 500 µL 85% acetonitrile twice, equipped with setting the vacuum at -0.05 bar. Samples were diluted with acetonitrile 1:5 (to 80% ACN) and loaded to pre-equilibrated cartridges. Solution was drawn through the cartridge, and the cartridges were washed twice with 600 µL 1:9:90 formic acid:water:acetonitrile solution. Fresh collection vials were then used to collect purified oligosaccharides. Oligosaccharides were eluted from the cartridges by washing twice with 50 µL water. The flow through was lyophilized using a vacuum concentrator and the samples were reconstituted in 25 µL of 5% acetonitrile with 0.1% formic acid in water and transferred to a suitable vial for LC analysis.

### Optimized Separation of Label-Free Oligosaccharides

Purified Oligosaccharides were separated using a Porous Graphite Carbon Column (HYPERCARB^{™} 2.1 x 100 mm, 3 µm from THERMO SCIENTIFIC^{™}) connected to a Waters Acquity UPLC^{™} system. Mobile Phase A and B were composed of 0.1% Formic Acid and 100% Acetonitrile, respectively. The column temperature was set to 60 °C and the flow rate was maintained at 0.3 mL/min. Samples were injected (5 µL volume) at 95% A, and separated using a gradient of 87% A to 78% A over 10 minutes, with a total run time of 30 min with equilibration. The Charged Aerosol Detector settings were 50 °C for the evaporation temperature, 1 for the power function, 25 Hz for data collection rate and a filter constant of 10 sec.

### Evaluating the extent of deglycosylation

In order to evaluate the extent of the deglycosylation reaction, time course studies were conducted for three different monoclonal antibodies, mAb A, mAb B and mAb C of different IgG subclasses. Samples were prepared according to the optimized procedure, and time points were taken and performed at t = 0 min, 5 min, 60 min, 240 min and 1440 min. For the non-rapid PNGase F treatment, samples were prepared identically to the rapid counterparts except that the reaction was carried out at 37 °C for 1440 min for the non-rapid PNGase F. These samples were then evaluated using reduced CE-SDS and the optimized chromatographic method to evaluate the extent of the deglycosylation reaction, to ensure that 60 min is sufficient to deglycosylate these molecules.

### Chromatography Optimization

Historically, glycan separation has been performed using hydrophilic interaction liquid chromatography (HILIC) because glycans are polar compounds which bind the amides present on the resin surface under high organic concentrations and can be eluted with increasing aqueous concentrations (Veillon et al., 2017). Typically, these separations have been performed with purified glycans that are labeled with a fluorescent tag such as 2-AA or 2-AB, which do impact binding and separation. In order to understand how the label-free glycans interacted with the columns, two HILIC HPLC columns (XBridge Glycan BEH Amide and PolyGLYCOPLEX A) were evaluated as a starting point. Mobile phase A was 100% acetonitrile and mobile phase B was 50 mM ammonium formate pH 4.4, typical mobile phase composition for HILIC separation. Both columns were evaluated using the non-labeled glycan standard library from Prozyme. The four major glycans (G0F, G1F, G2F and Man5) in the standard were observed for both columns, but poor resolution and weak signal limited the potential development for these columns. For both columns, shoulders were observed for the individual standard injections, something not previously observed using the fluorescent-labeled standards.

The next step was to evaluate UPLC-based HILIC columns, to see if resolution or peak shape could be improved. The UPLC HILIC columns (Acquity Glycan BEH Amide column and the TSKgel Amide column,) were screened with identical conditions as the HPLC experiments. The separation was superior for the UPLC system and BEH columns due to the higher resolving power available. Interestingly, the shoulders were still observed for the individual glycan standards, which was something that was unexpected at the onset of the study. The interaction between the HILIC stationary phase and the polar glycan analyte was further analyzed. When using HILIC for analysis, the released glycans are typically tagged with a fluorescent label on the free, reducing end. However, in this case, where no tag is used, this reducing end is exposed and can undergo an anomeric equilibrium (ref). These two species of the same glycan interact with the stationary phase in different ways depending if it is in the closed or open conformation, leading us to conclude that the shoulders are anomeric forms of the same glycan. After evaluating the available literature, it was decided that adding triethylamine to the mobile phase could help to force the equilibrium to one end, by increasing the pH of the solution (ref). However, when we used TEA in the mobile phase for HILIC, the glycans did not bind to the column because the pH modification was outside the functional range of HILIC column. This prompted us to evaluate reversed phase as an alternative, because the glycans were becoming more non-polar in the presence of TEA.

Four C18 columns were evaluated with no success (Peptide CSH, Acquity BEH, CORTECS C18+ and Kinetex C18). This was due to the fact that glycans aren't able to bind the C18 stationary phase as effectively as proteins or peptides can, and these columns are marketed for protein-based applications. A newer-to-market column was evaluated from Thermo, a porous graphite carbon (PGC) Hypercarb^{™} column. This column differs from typical reversed phase columns in that the stationary phase is composed of flat, hexagonally arranged carbon atoms which facilitate interactions between planar compounds (such as glycans). PGC provides the best isomer separation of glycans compared to any other stationary phase. Song et. al., 2015). Following manufacturer mobile phase recommendations, the initial screening yielded promising results, as the glycan standard library showed good resolution and signal intensity for all peaks when screening a very broad gradient (95% to 5%) of mobile phase A (0.1% TFA in water) and mobile phase B (acetonitrile). As the gradient was focused, the separation improved and no shoulder peaks were observed. It is likely that there are no shoulders on these glycan peaks because the mode of interaction is different, and the free, reducing end of the sugar does not make meaningful interactions with the stationary phase compared to how it does during HILIC separation.

The mobile phase and separation conditions were then optimized. Specifically, the ion-pairing reagent in the mobile phase was evaluated. Initially, Trifluoroacetic acid (TFA) and Triethylamine (TEA) were evaluated as ion-pairing reagents in the mobile phase. Separation under identical gradients were performed using mobile phase A with either 0.1% TFA or 0.1% TEA added. Higher resolution and better peak shape were observed when 0.1% TFA was added to the mobile phase compared to 0.1% TEA. Additionally, in the chromatograms from runs with TFA, additional glycan peaks were observed (judged as peaks not observed from mobile phase or assay blank injections) compared to runs with TEA. Adding 0.1% TFA to both mobile phases did not significantly improve separation compared to just adding it to mobile phase A, so the composition of the mobile phases was left at 0.1% TFA in water for mobile phase A and 100% acetonitrile for mobile phase B. The column temperature was evaluated at 60 and 70 °C, and it was determined that 70 °C provided better separation under these conditions. Finally, the gradient was optimized by comparing 95% to 75% A or 95% to 87%, then to 75% A. The resolution between minor peaks was better for the second gradient evaluated, so that was chosen as the optimal gradient.

### Re-optimization of Separation for MS compatibility

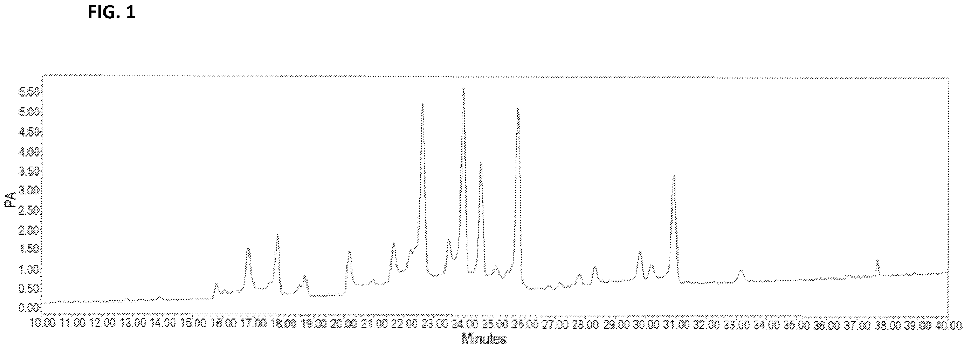

Because the glycans are unlabeled, a mass spectrometry-friendly method was developed. Five parameters were subsequently optimized: mobile phase composition (MPA and MPB), column temperature, separation time, and sample prep and injection volume. It is well understood that TFA suppresses mass spectrum signal, therefore it was desirable to evaluate formic acid as an ion-pairing reagent in place of TFA. This provided us an opportunity to re-develop the assay to further enhance separation performance and throughput. Preliminary comparison of mobile phase A (0.1% TFA or 0.1% FA) showed comparable results, which prompted us to further improve the separation efficiency. Next, adding 0.1% FA into mobile phase B (acetonitrile) was evaluated. A more stable baseline was observed as judged by less baseline drift, and the peak separation was better with formic acid in both mobile phases. Based on these results, it was concluded that 0.1% formic acid should be added to both mobile phase A (water) and mobile phase B (acetonitrile). The next optimization parameter was column temperature, and separations were performed at 50, 60, 70 and 80 °C. The results indicate that at 70 and 80 °C, the separation is much poorer than at 50 or 60 °C. At elevated temperature, many peaks are not resolved and tend to overlap with one another. By examining the peaks near the baseline, it was determined that 60 °C provided the best peak resolution throughout the chromatogram and was chosen as the optimized column temperature. The next parameter evaluated was the run time of the gradient from 87% to 78% mobile phase A, with 10, 20 and 30 min chosen for evaluation. The results strongly indicate that at 10 minutes, the separation is far superior compared to the longer gradients. At 10 min gradient time, more peaks are separated than at longer run times (FIG. 1).

The final parameter optimized was the deglycosylation conditions. The manufacturer's recommendation is to deglycosylate 100 µg of protein with 1 µL of rapid PNGase F for 5 minutes at 50°C. Early evaluation indicated that this was insufficient to obtain signal using CAD. After some preliminary optimization, 1 mg of protein was digested using 20 µL of 1:10 diluted Rapid PNGase F for 60 minutes. To evaluate the extent of the deglycosylation, reactions were set up for 60 min, along with a control of non-rapid PNGase F at 24 hours. These reactions were then analyzed using CE-SDS (reduced with the non-deglycosylated sample control) and with the optimized glycan method. The CE-SDS data indicates that after 60 minutes, more than 98% of the protein is deglycosylated, as judged by the disappearance of the main peak on non-reduced and the disappearance of the heavy chain peak on the reduced, and formation of non-glycosylated main peak/heavy chain in the respective electropherograms. The separation using the glycan method also supported these findings, with the signal intensity reaching a plateau after 60 minutes. To ensure maximum deglycosylation, 60 minutes was chosen, as both CE-SDS and chromatography results showed completion after 60 minutes.

### EXAMPLE 2

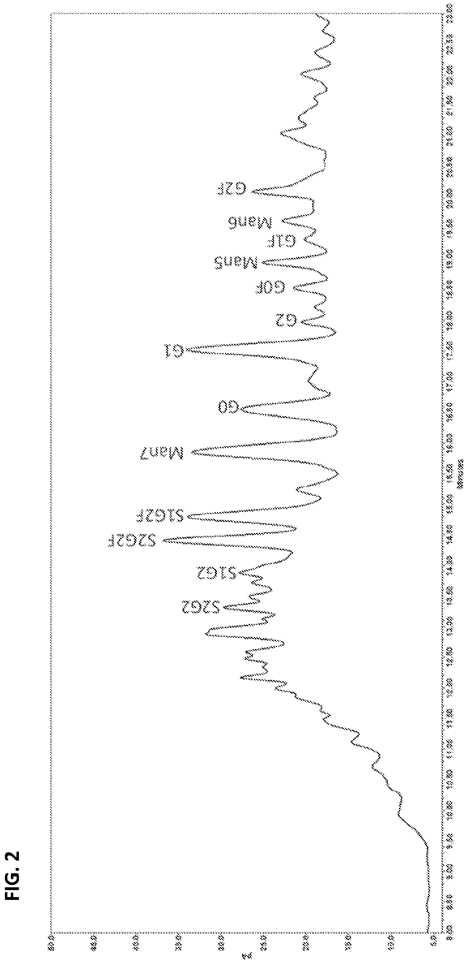

Oligosaccharides were released and analyzed from mAb samples according to Example 1, with the following changes to the mobile phase conditions: Mobile phase A: 0.1% triethylamine (TEA) in water; and mobile phase B: 0.1% triethylamine (TEA) in acetonitrile (FIG. 2). The other experiments were repeated as shown in Example 1. The sample glycan chromatogram produced from a separation analysis is shown in FIG. 2.

## Claims

1. A label-free method of quantifying a glycosylation profile of a recombinant protein, comprising:
(a) releasing one or more N-linked glycans from the recombinant protein by an enzyme prior to analysis;
(b) separating the released N-linked glycans using a chromatography column; and
(c) measuring the separated N-linked glycans by ELSD, NQAD, refractive index detector, or a charged aerosol detector (CAD).

2. The method of claim 1, wherein the recombinant protein is at least about 80%, at least about 85%, at least about 90%, at least about 95%, at least about 96%, at least about 97%, at least about 98%, at least about 99%, or about 100% pure.

3. The method of claim 1 or 2, wherein the column is a mixed mode column.

4. The method of any one of claims 1 to 3, wherein the column is a mixed mode porous graphite carbon (PGC) column.

5. The method of any one of claims 1 to 4, wherein the enzyme comprises peptide N-glycosidase F (PNGaseF).

6. The method of any one of claims 1 to 5, wherein the one or more N-linked glycans that are separated are measured by a charged aerosol detector (CAD).

7. The method of any one of claims 1 to 6, wherein the chromatography comprises a first mobile phase and a second mobile phase, wherein the first mobile phase and the second mobile phase are different.

8. The method of claim 7, wherein the first mobile phase comprises formic acid (FA), Trifluoroacetic acid (TFA), Triethylamine (TEA), or any combination thereof.

9. The method of claim 7 or 8, wherein the second mobile phase comprises formic acid (FA), Trifluoroacetic acid (TFA), Triethylamine (TEA), or any combination thereof.

10. The method of any one of claims 1 to 9, wherein the separation is performed at a temperature lower than 70 °C.

11. The method of claim 10, wherein the temperature is between about 50 °C and about 70 °C, about 50 °C and about 60 °C, about 60 °C and about 70 °C, about 55 °C and about 65 °C, about 55 °C and about 60 °C, about 60 °C and about 65 °C, about 65 °C and about 70 °C, about 50 °C and about 55 °C.

12. The method of any one of claims 1 to 11, wherein the one or more N-glycans are Galactose (Gal), N-Acetylgalactosamine (GalNAc), Galactosamine (GalN), Glucose (Glc), N-Acetylglucosamine (GlcNAc), Glucosamine (GlcN), Mannose (Man), N-Acetylmannosamine (ManNAc), Mannosamine (ManN), Xylose (Xyl), N-Acetylneuraminic acid (Neu5Ac), N-Glycolylneuraminic acid (Neu5Gc), 2-Keto-3-deoxynononic acid (Kdn), Fucose (Fuc), Glucuronic Acid (GlcA), Iduronic acid (IdoA), Galacturonic acid (GalA), Mannuronic acid (ManA), or any combination thereof.

13. The method of any one of claims 1 to 12, wherein the recombinant protein is an antibody.

14. The method of claim 13, wherein the antibody is an anti-GITR antibody, an anti-CXCR4 antibody, an anti-CD73 antibody, an anti-TIGIT antibody, an anti-OX40 antibody, an anti-LAG3 antibody, an anti-CSF1R antibody, or an anti-IL8 antibody.

15. The method of any one of claims 1 to 14, wherein the recombinant protein is a fusion protein.

## Patentansprüche

1. Markierungsfreies Verfahren zur Quantifizierung eines Glykosylierungsprofils eines rekombinanten Proteins, umfassend:
(a) Freisetzen eines oder mehrerer N-gebundener Glykane aus dem rekombinanten Protein durch ein Enzym vor der Analyse;
(b) Trennen der freigesetzten N-gebundenen Glykane unter Verwendung einer Chromatographiesäule; und
(c) Messen der abgetrennten N-gebundenen Glykane durch ELSD, NQAD, einen Brechungsindexdetektor oder einen geladenen Aerosoldetektor (CAD).

2. Verfahren nach Anspruch 1, wobei das rekombinante Protein mindestens etwa 80 %, mindestens etwa 85 %, mindestens etwa 90 %, mindestens etwa 95 %, mindestens etwa 96 %, mindestens etwa 97 %, mindestens etwa 98 %, mindestens etwa 99 % oder etwa 100 % rein ist.

3. Verfahren nach Anspruch 1 oder 2, wobei die Säule eine Mischmodus-Säule ist.

4. Verfahren nach einem der Ansprüche 1 bis 3, wobei es sich bei der Säule um eine Säule mit porösem Graphitkohlenstoff (PGC) im Mischmodus handelt.

5. Verfahren nach einem der Ansprüche 1 bis 4, wobei das Enzym Peptid-N-Glycosidase F (PNGaseF) umfasst.

6. Verfahren nach einem der Ansprüche 1 bis 5, wobei das eine oder die mehreren N-gebundenen Glykane, die abgetrennt werden, mit einem geladenen Aerosoldetektor (CAD) gemessen werden.

7. Verfahren nach einem der Ansprüche 1 bis 6, wobei die Chromatographie eine erste mobile Phase und eine zweite mobile Phase umfasst, wobei die erste mobile Phase und die zweite mobile Phase unterschiedlich sind.

8. Verfahren nach Anspruch 7, wobei die erste mobile Phase Ameisensäure (FA), Trifluoressigsäure (TFA), Triethylamin (TEA) oder eine Kombination davon umfasst.

9. Verfahren nach Anspruch 7 oder 8, wobei die zweite mobile Phase Ameisensäure (FA), Trifluoressigsäure (TFA), Triethylamin (TEA) oder eine Kombination davon umfasst.

10. Verfahren nach einem der Ansprüche 1 bis 9, wobei die Trennung bei einer Temperatur von weniger als 70 °C durchgeführt wird.

11. Verfahren nach Anspruch 10, wobei die Temperatur zwischen etwa 50 °C und etwa 70 °C, etwa 50 °C und etwa 60 °C, etwa 60 °C und etwa 70 °C, etwa 55 °C und etwa 65 °C, etwa 55 °C und etwa 60 °C, etwa 60 °C und etwa 65 °C, etwa 65 °C und etwa 70 °C, etwa 50 °C und etwa 55 C° liegt.

12. Verfahren nach einem der Ansprüche 1 bis 11, wobei das eine oder die mehreren N-Glykane Galactose (Gal), N-Acetylgalactosamin (GalNAc), Galactosamin (GalN), Glucose (Glc), N-Acetylglucosamin (GlcNAc), Glucosamin (GlcN), Mannose (Man), N-Acetylmannosamin (ManNAc), Mannosamin (ManN), Xylose (Xyl), N-Acetylneuraminsäure (Neu5Ac), N-Glycolylneuraminsäure (Neu5Gc), 2-Keto-3-desoxynononsäure (Kdn), Fucose (Fuc), Glucuronsäure (GlcA), Iduronsäure (IdoA), Galacturonsäure (GalA), Mannuronsäure (ManA) oder eine Kombination davon sind.

13. Verfahren nach einem der Ansprüche 1 bis 12, wobei das rekombinante Protein ein Antikörper ist.

14. Verfahren nach Anspruch 13, wobei der Antikörper ein Anti-GITR-Antikörper, ein Anti-CXCR4-Antikörper, ein Anti-CD73-Antikörper, ein Anti-TIGIT-Antikörper, ein Anti-OX40-Antikörper, ein Anti-LAG3-Antikörper, ein Anti-CSF1R-Antikörper oder ein Anti-IL8-Antikörper ist.

15. Verfahren nach einem der Ansprüche 1 bis 14, wobei das rekombinante Protein ein Fusionsprotein ist.

## Revendications

1. Procédé de quantification sans marqueur d'un profil de glycosylation d'une protéine recombinante, comprenant :
(a) la libération d'un ou plusieurs glycanes N-liés de la protéine recombinante par un enzyme avant une analyse,
(b) la séparation des glycanes N-liés libérés en se servant d'une colonne chromatographique, et
(c) la mesure des glycanes N-liés séparés par un ELSD, un NQAD, un détecteur d'indice de réfraction ou un détecteur d'aérosols chargés (CAD).

2. Procédé selon la revendication 1, dans lequel la protéine recombinante est pure à au moins environ 80 %, à au moins environ 85 %, à au moins environ 90 %, à au moins environ 95 %, à au moins environ 96 %, à au moins environ 97 %, à au moins environ 98 %, à au moins environ 99 %, ou à environ 100 %.

3. Procédé selon la revendication 1 ou 2, dans lequel la colonne est une colonne en mode mixte.

4. Procédé selon l'une quelconque des revendications 1 à 3, dans lequel la colonne est une colonne de carbone graphite poreux (PGC) en mode mixte.

5. Procédé selon l'une quelconque des revendications 1 à 4, dans lequel l'enzyme comprend une peptide-N-glycosidase F (PNGaseF).

6. Procédé selon l'une quelconque des revendications 1 à 5, dans lequel les un ou plusieurs glycanes N-liés qui sont séparés sont mesurés par un détecteur d'aérosols chargés (CAD).

7. Procédé selon l'une quelconque des revendications 1 à 6, dans lequel la chromatographie comprend une première phase mobile et une deuxième phase mobile, dans lequel la première phase mobile et la deuxième phase mobile sont différentes.

8. Procédé selon la revendication 7, dans lequel la première phase mobile comprend de l'acide formique (FA), de l'acide trifluoroacétique (TFA), de la triéthylamine (TEA) ou une combinaison quelconque de ceux-ci.

9. Procédé selon la revendication 7 ou 8, dans lequel la deuxième phase mobile comprend de l'acide formique (FA), de l'acide trifluoroacétique (TFA), de la triéthylamine (TEA) ou une quelconque combinaison de ceux-ci.

10. Procédé selon l'une quelconque des revendications 1 à 9, dans lequel la séparation est exécutée à une température inférieure à 70 °C.

11. Procédé selon la revendication 10, dans lequel la température est entre environ 50 °C et environ 70 °C, environ 50 °C et environ 60 °C, environ 60 °C et environ 70 °C, environ 55 °C et environ 65 °C, environ 55 °C et environ 60 °C, environ 60 °C et environ 65 °C, environ 65 °C et environ 70 °C, entre environ 50 °C et environ 55 °C.

12. Procédé selon l'une quelconque des revendications 1 à 11, dans lequel les un ou plusieurs N-glycanes sont du galactose (Gal), de la N-acétylgalactosamine (GalNAc), de la galactosamine (GalN), du glucose (Glc), de la N-acétylglucosamine (GlcNAc), de la glucosamine (GlcN), du mannose (Man), de la N-acétylmannosamine (ManNAc), de la mannosamine (ManN), du xylose (Xyl), de l'acide acétylneuraminique (Neu5Ac), de l'acide N-glycolylneuraminique (Neu5Gc), de l'acide 2-céto-3-désoxynononique (Kdn), du fucose (Fuc), de l'acide glucuronique (GlcA), de l'acide iduronique (IdoA), de l'acide galacturonique (GalA), de l'aide mannuronique (ManA) ou une quelconque combinaison de ceux-ci.

13. Procédé selon l'une quelconque des revendications 1 à 12, dans lequel la protéine recombinante est un anticorps.

14. Procédé selon la revendication 13, dans lequel l'anticorps est un anticorps anti-GITR, un anticorps anti-CXCR4, un anticorps anti-CD73, un anticorps anti-TIGIT, un anticorps anti-OX40, un anticorps anti-LAG3, un anticorps anti-CSF1R ou un anticorps anti-IL8.

15. Procédé selon l'une quelconque des revendications 1 à 14, dans lequel la protéine recombinante est une protéine de fusion.
